(19) Europäisches Patentamt
European Patent Office
Office européen des brevets

(11) **EP 3 676 852 B1**

(12) **EUROPEAN PATENT SPECIFICATION**

(45) Date of publication and mention
of the grant of the patent:
**10.07.2024 Bulletin 2024/28**

(21) Application number: **18783166.4**

(22) Date of filing: **04.09.2018**

(51) International Patent Classification (IPC):
**G16H 40/20** *(2018.01)*   **G16H 50/30** *(2018.01)*
**G16H 50/70** *(2018.01)*   **G16H 20/40** *(2018.01)*
**G16H 20/10** *(2018.01)*

(52) Cooperative Patent Classification (CPC):
**G16H 50/30; G16H 20/10; G16H 20/40;
G16H 40/20; G16H 50/70**

(86) International application number:
**PCT/US2018/049381**

(87) International publication number:
**WO 2019/046854 (07.03.2019 Gazette 2019/10)**

(54) **SYSTEM, METHOD, COMPUTER PROGRAM PRODUCT AND APPARATUS FOR DYNAMIC PREDICTIVE MONITORING IN THE CRITICAL HEALTH ASSESSMENT AND OUTCOMES STUDY/SCORE/(CHAOS)**

SYSTEM, VERFAHREN, COMPUTERPROGRAMMPRODUKT UND -VORRICHTUNG ZUR DYNAMISCHEN PRÄDIKTIVEN ÜBERWACHUNG BEI DER KRITISCHEN GESUNDHEITSBEWERTUNG UND ERGEBNISSE DER STUDIENERGEBNISSE / (CHAOS)

SYSTÈME, MÉTHODE, PRODUIT DE PROGRAMME D'ORDINATEUR ET APPAREIL POUR LA SURVEILLANCE PRÉDICTIVE DYNAMIQUE DANS L'ÉVALUATION DE LA SANTÉ CRITIQUE ET LE SCORE DE L'ÉTUDE DES RÉSULTATS (CHAOS)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.09.2017   US 201762553490 P**

(43) Date of publication of application:
**08.07.2020 Bulletin 2020/28**

(73) Proprietor: **University of Cincinnati
Cincinnati, OH 45206-0829 (US)**

(72) Inventor: **DEMAZUMDER, Deeptankar
Cincinnati
OH 45221-0829 (US)**

(74) Representative: **Sadler, Peter Frederick
Reddie & Grose LLP
The White Chapel Building
10 Whitechapel High Street
London E1 8QS (GB)**

(56) References cited:
**WO-A2-2013/003787     US-A1- 2015 133 795**

• **ANONYMOUS: "Marginal likelihood - Wikipedia", 9 December 2016 (2016-12-09), XP055523177, Retrieved from the Internet <URL:https://en.wikipedia.org/w/index.php?title=Marginal_likelihood&oldid=753887384> [retrieved on 20181113]**

**Description**

## BACKGROUND OF THE INVENTION

It is nearly a universal truth in medical treatment that early diagnosis of illness, leading to early therapy, improves clinical outcomes. While risk scoring systems, e.g., APACHE, SAPS, and the Seattle Heart Failure risk score, have improved allocation of resources toward those cases requiring early intervention, they require data from multiple sources and cannot predict individual mortality risk. Diseases such as sepsis, cardiac arrhythmias, seizures, pulmonary embolism, shock, cardiac arrest, stroke, heart failure, and respiratory failure have rapid onset and evolution, and require immediate individualized intervention to prevent impending morbidity and mortality.

With annual intensive care unit (ICU) admission rates of about 4 million per year in the United States and mortality rates of 14-20%, i.e., 600,000-800,000 deaths, ICU patients are particularly vulnerable to subacute, potentially catastrophic complications. Many of these complications, however, may be amenable to early therapy. For example, the likelihood of mortality for patients with septic shock increases by 7.6% per hour that antibiotic therapy is delayed.

Further, ventricular tachycardia and fibrillation (VT/VF) are lethal cardiac arrhythmias, claiming a quarter million lives per year in the United States from sudden cardiac death (SCD). The identification of individuals with the highest risk for SCD poses a great clinical challenge and socioeconomic burden. Patients with heart failure are at a higher risk and implantable cardioverter-defibrillators (ICDs) prevent SCD in these patients by delivering shocks for VT/VF. However, more than half a million patients per year are eligible for ICDs in the United States based on heart failure alone but most of them do not experience VT/VF requiring shocks for VT/VF. Most episodes of SCD occur in individuals without heart failure who have no significant clinical signs of this impending catastrophic event.

In individuals with these clinical and subclinical illnesses, early diagnosis leading to early therapy can improve their outcomes. The need for a readily available and efficient system, method and apparatus for risk stratification of individual patients is paramount and a clinical, research and public health priority.

Although ICU patients are heavily instrumented, the technological methodology to capture, structure, secure, store and analyze robust physiological time-series data has been lacking. Further, the sheer amount and complexity of these massive data sets are beyond cognitive assimilation and analytical synthesis. Further, physiological data are more readily available from multiple sources such as wearable electronics and home monitors. Little progress has been made on how to extract meaningful information from these data to impact patient care.

The inventor has previously published work in conjunction with the Johns Hopkins University School of Medicine describing hardware and software environments that can implement continuous acquisition, annotation and storage of physiological waveform data (e.g., from ICU telemetry monitors), connect that data with an electronic health record (EHR), and perform analyses of the data using real-time stream-computing and high-performance computing systems. Further, the inventor has developed an open-source, open-platform informatics and analytics infrastructure for the research community for studying animal and human physiological waveforms during periods of health, critical illness and after recovery from illness. These prior studies have proposed that repetition of distinct non-linear patterns underlying the variability of physiological signals reflects altered homeostatic signaling and provides unique prognostic insight. These non-linear signatures of subclinical deterioration may be indistinguishable in conventional clinical measures but revealed via complex informatic methods. This work is described in PCT Publication PCT/US2013/040751, PCT/US2013/040677, and US Patent Publication 2015/0133795 filed by Gordon F. Tomaselli and the inventor herein.

In this context, a human organism may be modeled as a complex network of interconnected molecules, cells, tissues and organs that communicate with each other via afferent and efferent neuro-axial and other signaling processes. The degree of coupling within and between prominent network components is altered in illness. Physiological signatures reflect dynamic integrative aspects of inter-organ communications and the evolution of collective network derangements in response to brewing subclinical critical illness.

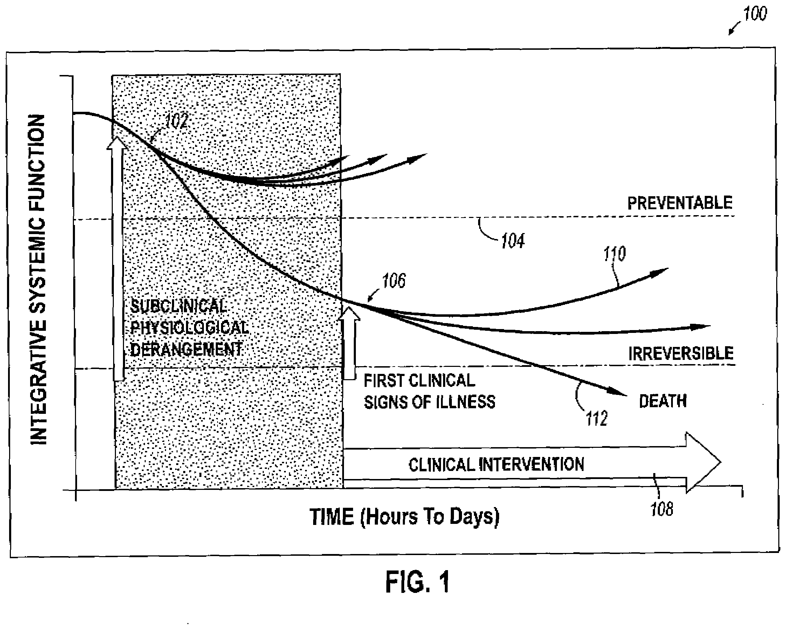

By way of illustration, Fig. 1 illustrates the typical timeline 100 of impairment of an integrative systemic function over development, detection and intervention of critical illness. The earliest period 102 of derangement is identified as subclinical because conventional clinical measures, static physiological measures (e.g., average heart rate), and aggregate-based risk scores are limited for detecting these signatures, for example because virtually all biosignals demonstrate non-linear properties. It is believed that many illnesses are entirely preventable if the systemic derangement can be identified before it crosses the preventable threshold 104. By the period 106 when clinical signs of illness appear, a patient may or may not be in a reversible condition. Some patients may receive intervention 108 in time to take a recovery path 110, others may not be recoverable and may die 112.

For example, analyses of beat-to-beat changes in features of continuous electrocardiography (ECG) recordings have been commonly used to gauge disease processes, i.e., low heart rate variability (HRV) indicates pathology. Complexity analyses of beat-to-beat changes in heart rate, one of the prominent network components, have proven important for early therapy and reduced mortality in recent clinical trials of sepsis in the neonatal ICU. The cholinergic anti-inflammatory pathway provides a mechanistic basis for how the systemic inflammatory response syndrome, which precedes sepsis and septic shock, can be reflected by heart rate dynamics.

Continuous predictive monitoring based on dynamic analysis of time-varying physiological signals identifies "hidden" signatures of subclinical deterioration that are indistinguishable in conventional clinical measures. These signatures provide unique prognostic insight. Extending this paradigm to hospitalized patients and non-hospitalized asymptomatic individuals can provide unique prognostic insight into their function, health and risk of impending adverse events over the acute, subacute or longer term, and thus, can save many lives. WO 2013/003787 A2 describes a system and method for determining a patient's susceptibility to develop cardiorespiratory instability wherein physiological parameters are analyzed with respect to a dynamics systems model to produce and indicator associated with a probability that the patient will become unstable.

US 2015/133795 A1 describes a system and method for the assessment of health and mortality based on dynamic nonlinear calculations of self-similar fluctuation patterns in a time series of QT intervals and of other physiological signals, such as RR intervals, temperature, blood pressure, respiration, saturation of peripheral oxygen, intracardiac pressures, and electroencephalogram. In order to nonlinearly determine health and mortality, time series of QT intervals and of other physiological signals (e.g.. RR intervals) are simultaneously obtained, and entropy values are calculated for each signal over the same temporal interval. "EntropyX" is calculated from relative changes between moments and entropy of QT intervals and those of other physiological signals over seconds to days. The absolute and relative entropy values at a specific time point and/or subsequent changes in entropy over future time points can be used to determine a treatment plan for the subject.

## SUMMARY OF THE INVENTION

The present invention provides, broadly, a method for assessing the likelihood of an individual for experiencing a condition (which may be an illness, an improvement from illness, or an improvement in athletic conditioning) by obtaining one or more time-varying signals from continuous monitoring of the individual or the individual's environment, processing the signals to develop signatures for those signals, and calculating an integrated likelihood of experiencing the condition which can be provided to an individual and/or a trainer, supervising authority, or health-care provider for the individual, and updated on a time schedule.

In the described particular environment, the integrated likelihood is computed by calculating a set of parameters from time-varying signals using linear and non-linear analyses of a signal and/or features derived from at least one of the signals, which may comprise one of many time-varying physiological and/or environmental signals. Candidate signals include electrocardiogram (ECG), electroencephalogram (EEG), electrooculogram (EOG), electromyogram (EMG), thoracic and/or abdominal excursions recorded by inductive plethysmography bands, recordings of nasopharyngeal airflow, pulse oximetry, intracardiac pressure, intracardiac electrogram (EGM), temperature, gauge and/or camera-sensed body position, sleep/activity monitor, acid/base monitoring, respiration, acceleration or ambulation monitor, ambient light exposure monitor, ambient sound exposure monitor, ambient temperature monitor, invasive and/or noninvasive blood pressure readings, glucose level monitor readings, hemoglobin oxygen saturation monitor readings, arterial blood oxygen and carbon dioxide partial pressure monitor readings, tissue hemoglobin oxygen saturation readings, nasopharyngeal airflow, and metabolic activity based on one or more of expired $CO_2$ and inspired $O_2$, respiration rate, respiration volume, and laboratory measurements such as plasma count and blood lipid density concentrations.

In addition to the quantitative data described above, embodiments of the invention may also gather qualitative data such as individual, family, and/or health care provider subjective reporting of a relative status of the individual's condition.

In the described embodiment, signals representing continuous monitoring over a time frame of minutes to days are processed to create an integrated likelihood measure which is updated on a time schedule. Further, a profile of the individual comprising a composite of the individual's time-varying signatures and integrated likelihood of groups of individuals with similar time-varying signatures is created, stored, and used in the individual's subsequent treatment or training.

The invention is particularly applicable to assessment of persons for pre-clinical identification of impending conditions such as critical heart failure, organ failure, death and the like before those persons are symptomatic and even before they are hospitalized.

Furthermore, the invention is usable as a dynamic personal health and fitness assistant, by developing data from monitors such as wearable devices, transportable monitors, stationary exercise machine or rest condition monitors, time-scheduled monitors, and combinations thereof. Data regarding demographics, training regimens, nutrition and general activity, and any adverse or undesired event(s) that occur during the monitoring period, can be included in the predictive modeling to optimize, evolve and tailor an individual's course of training or activity or diet to improve personal health and athletic performance.

The invention has found a particular correlation of signal variations during sleep onset or a sleep state transition to subsequent health outcomes and can therefore be an important addition to sleep assessment therapies. Development

EP 3 676 852 B1

of signatures for such applications can involve comparison of signals from an individual in different sleep states or during progression between sleep states, and by comparison of signatures of a plurality of subjects diagnosed with a pathological condition such as heart failure to signatures of a plurality of normal subjects when monitored during sleep periods.

The invention is further applicable in the collection and analysis of treatment information for assessment of treatment quality for an individual and improvement of treatment methods. In this application, one or more time-varying physiological or environmental signals associated with an individual are collected along with medical treatment information for the individual including one or more of pharmacological treatments, surgical or non-surgical caregiver activity involving the individual, and qualitative assessments of the individual's well-being from caregivers, the individual or observers. A signature profile of the signals and medical treatment information is created for each of a plurality of individuals and series of time periods, and these are compared to an individual's data indicating subsequent outcomes, disease states or conditions, in order to calculate a quality measure of the individual's care indicating the efficacy or lack thereof of interventions identified in the intervention information such as but not limited to medical treatments identified in the medical treatment information.

The above and other objects and advantages of the present invention shall be made apparent from the accompanying drawings and the description thereof.

## BRIEF DESCRIPTION OF THE DRAWINGS

Fig. 1 is an illustration of the preventability of illness over time;

Fig. 2 illustrates a hardware environment applicable to the invention;

Fig. 3 is a flow chart of a processing method used in accordance with the present invention in evaluating physiological signals;

Fig. 4 is an example of a mortality risk display of the kind which may be presented with other physiological data for hospitalized or non-hospitalized individual;

Fig. 5 illustrates a data flow for a method of processing data to evaluate condition, athletic status or health care quality and predict outcomes in accordance with the present invention;

Fig. 6 illustrates physiological signals which may be used in accordance with present invention in evaluating physiological condition, athletic status, or health care quality;

Fig. 7 illustrates ECG landmarks, used in one particular embodiment of the present invention;

Fig. 8 illustrates distinctive features of pulse oximetry photoplethysmography (PPG) waveform for illustration of this particular embodiment;

Fig. 9 illustrates the derivation of mean volume pulse amplitude in a PPG signal in this particular embodiment;

Figs. 10A, 10B and 10C illustrate details of combined recording and analysis of ECG and PPG signal complex in an individual;

Fig. 11 is a flow chart of the signal analysis used in the particular illustrated embodiment;

Fig. 12 is an illustration of prognostic insights obtained with the use of EntropyX$_{QT}$ on a community of asymptomatic ostensibly healthy persons in comparison to patients with congestive heart failure (CHF);

Fig. 13 is an illustration of EntropyX$_{QT}$ characteristics observed before, during and after sleep onset of asymptomatic community adults who lived through the subsequent experimental follow up period of up to 10 years, compared to those who died during that period;

Fig. 14 is an illustration of sympathovagal balance characteristics observed before, during and after sleep onset of asymptomatic community adults who lived through the subsequent experimental follow up period of up to 10 years, compared to those who died during that period;;

4

Fig. 15 is an illustration of entropy rate calculations applied to serum fractionated lipoprotein measurements in a population of men and women with and without diabetes, illustrating that unique prognostic insight can be gained by applying this novel method to routine laboratory analysis of body fluids of men and women in the absence and presence of known risk factors;

Fig. 16 is an illustration of EntropyXQT measurements performed on 24-hour ECGs of guinea pigs with heart failure showing that this measurement predicts animal mortality over the subsequent follow up period with a prognostic value similar to that in humans;

Fig. 17 is an illustration of data produced in a multicenter prospective observational study of 851 heart failure patients showing EntropyXQT measurements and its strong and independent prediction of SCD and mortality over the subsequent 21-69 months.

The accompanying drawings, which are incorporated in and constitute a part of this specification, illustrate embodiments of the invention and, together with a general description of the invention given above, and the detailed description of the embodiments given below, serve to explain the principles of the invention.

## DETAILED DESCRIPTION OF THE INVENTION

The present invention relates generally to a method, system, computer program product and apparatus for individualized health evaluation, risk stratification and early identification of impending catastrophic or worsening illness or other adverse events, or alternatively for assessment of athletic condition and functional capacity, or assessment of health care quality, based on analysis of time-varying physiological signals using informatic methods. The informatic methods include control-theoretic, mechanistic model- and machine-learning based algorithm development and deployment in a device, apparatus or computing platform, linear and nonlinear analyses, utilizing hardware and software tools for data acquisition, storage, management and analysis of electronic health records (EHR), development of metadata standards, ontology, and annotation (data catalogs); data visualization; and predictive modeling.

While prior work has indicated that ECG-derived heart rate dynamics alone have prognostic value for predicting onset of serious illness, in accordance with the present invention, continuous analysis of other features derived from ECG waveforms and other time-varying physiological processes are also monitored over the course of an individual's health, illness, response to therapy, athletic performance, activities of daily living and/or experience of adverse or undesired event(s) to tailor a predictive model unique to an individual, and by so doing the invention achieves an even higher order of information retrieval and automated evolution that markedly increases this predictive accuracy in the individual, and more broadly, in groups comprised of the individual (e.g., gender, ethnicity, preexisting disease).

By integrating approaches from the physical, biological, computational, statistical and clinical sciences, dynamic predictive monitoring according to the present invention complements conventional clinical predictors and provides unique prognostic insight into an individual's acute, sub-acute, and/or long-term probability risk for health or illness before and during critical illness. This inventive method can then be used to risk-stratify individuals, identify individuals in need for medical care and to guide precision (personalized) medical care for an individual, including, but not limited to, therapy, e.g., in an asymptomatic, otherwise healthy-appearing person, or in a symptomatic patient with pre-existing mild, moderate, advanced, or critical illness. Further, the method can evaluate athletic condition in an individual seeking enhancement of athletic capability. Finally, the method can evaluate the quality of care and performance of particular caregivers.

Dynamic predictive monitoring in accordance with the present invention incorporates linear and non-linear time series analyses of any or all available time-varying physiological signals, including, but not limited to, EEG, EOG, EMG, thoracic and/or abdominal excursions recorded by inductive plethysmography bands, recordings of nasopharyngeal airflow, pulse oximetry, intracardiac pressures, gauge and/or camera-sensed body position, sleep/activity monitor, acid/base monitoring, respiration, acceleration or ambulation monitor, ambient light exposure monitor, ambient sound exposure monitor, ambient temperature monitor, invasive and/or noninvasive blood pressure readings, glucose level monitor readings, hemoglobin oxygen saturation monitor readings, arterial blood oxygen and carbon dioxide partial pressure monitor readings, tissue hemoglobin oxygen saturation readings, metabolic activity based on one or more of expired $CO_2$ and inspired $O_2$, respiration rate, respiration volume, laboratory measurements such as plasma count and blood lipid density concentrations, intraarterial and noninvasive blood pressure measurements, intracardiac pressures, hemoglobin $O_2$ saturation, partial pressure of arterial blood $O_2$ and $CO_2$, near infrared spectroscopy measurements of tissue hemoglobin $O_2$ saturation, EGM and ECG measures (e.g., QT, RR, QRS, JT, PR intervals). Further, linear deterministic and nonlinear measures of comparison between the different physiological time series may also be incorporated into the predictive models.

Dynamic predictive monitoring may be used for early identification of underlying disease processes to implement early, more effective therapy, and also for repeated measures of risk over the course of illness and/or response to therapy. Dynamic monitoring may also be used to assess the health status or athletic condition of a person not at risk for illness. Thus, the analysis of short-term repetitive patterns underlying the variability of time-varying physiological signals, as well as the analysis of long-term patterns over a longer time scale, i.e., during the course of illness and/or response to therapy or training, will provide unique diagnostic, therapeutic and prognostic insight, resulting in improved clinical or training decisions and management. Furthermore, by combining physiological information with data gathered from a healthcare provider, the quality of a provider's intuition and perception of patient status can also be evaluated and graded, for example as part of a training or intern program.

Whereas current clinical practice uses statistical moments, e.g., mean and standard deviation of time-varying physiological signals, to guide clinical decisions and management, physiological and pathophysiological processes in vivo are continuous time-varying phenomena that are non-stationary and not at equilibrium. Virtually all biological signals demonstrate nonlinear properties, e.g., drift in heart rate or blood pressure during sleep-wake cycles. Although linear, deterministic analyses require the assumption of stationarity of physiological signals, they may provide useful measures by using some arbitrary criteria to judge stationarity. However, important information on pathological states and natural physiological processes contained within nonstationary properties are lost. Thus, non-linear analyses complement linear deterministic analyses by identifying complex patterns underlying the variability of physiological signals and providing unique insight into physiological, pathophysiological and therapeutic processes that increases prognostic value.

A method, system, computer program product and apparatus of an embodiment of the present invention may be implemented using hardware, software, algorithm, or a combination thereof, in one or more computer or other processing systems, in which high resolution time-varying physiological signals are obtained from an individual to mathematically characterize signatures of health and illness. Unlike the common practice of using low resolution physiological data or static aggregate physiological measures that are readily available, implanting methods for the acquisition and analysis of high resolution signals are important for avoiding artifacts and markedly improve the accuracy and precision of signature characterization. Further, signatures of certain illnesses are optimally detected through analyses of specific linear and non-linear characteristics, phase and time lags, temporal window lengths, degree of coupling between different physiological signals, and their changes over time. These signatures are determined from large databases of physiological time series obtained from representative individuals of health and illness, as well as from those obtained in real time.

For example, repetition of distinct patterns underlying the variability of time-varying physiological signals provides unique prognostic insight that is not identifiable in conventional clinical measures. The signatures obtained from one or more time series of different physiological measures from the same individual at one or more time points are used to construct new, or enhance pre-existing, multivariate mathematical and statistical predictive models. In turn, these models provide an individual's acute, sub-acute, and/or long-term probability risk for health or illness. By integrating approaches from the physical, biological, computational, statistical and clinical sciences, this information can then be used to risk-stratify individuals, identify individuals in need for medical care and to guide precision (personalized) medical care for an individual, including but not limited to diagnostic assessment and therapy in asymptomatic, otherwise healthy-appearing persons with little or no apparent illness, evaluation of symptomatic patients with pre-existing mild, moderate or advanced illness, and evaluation of persons undergoing a training or fitness regimen to evaluate its effectiveness.

Unlike the common practice of measuring risk predictors at discrete time points (e.g., at first presentation or sign of illness), the present invention utilizes novel artificial intelligence (AI) algorithms at multiple levels for dynamic predictive monitoring (DPM) that ingest large amounts of continuous physiological data to execute automated tasks, facilitate early recognition of anomalous trends, and support complex clinical or other management decisions. The "smart" algorithms iteratively compare predicted health / predicted risk with later-determined ultimate adverse or undesired outcomes to achieve an even higher order of information retrieval and automated evolution, progressively improving upon their ability to identify signatures of "hidden" subclinical critical illness or heath data in an individual and, more broadly, in groups (e.g., gender, ethnicity, disease). Further, unique signatures of health and illness are stored and compared over an individual's follow-up or hospital readmission course to further refine its predictive ability and to enhance the delivery of personalized healthcare.

The fundamental precepts of the present invention include: (1) Impending catastrophic adverse events have subclinical phases during which early diagnosis and treatment have lifesaving effects, as presented in Fig. 1; (2) Signatures of early subclinical illness or improvement in health are comprised of distinct patterns "hidden" within otherwise normal appearing body signals; (3) These patterns reflect progressive uncoupling or recoupling of highly complex but adaptive homeostatic signaling as well as autonomic, humoral and cytokine modulation of organs, such as the brain, heart and lungs; and (4) Implementation of an infrastructure for extracting decision support from waveform biosignals using validated algorithms and feeding the calculated data back to the individual, attending physician or trainer, and/or to instrumentation accessible to them (bedside instruments, electronic health records, wearable devices or other feedback systems) can test its impact on outcomes.

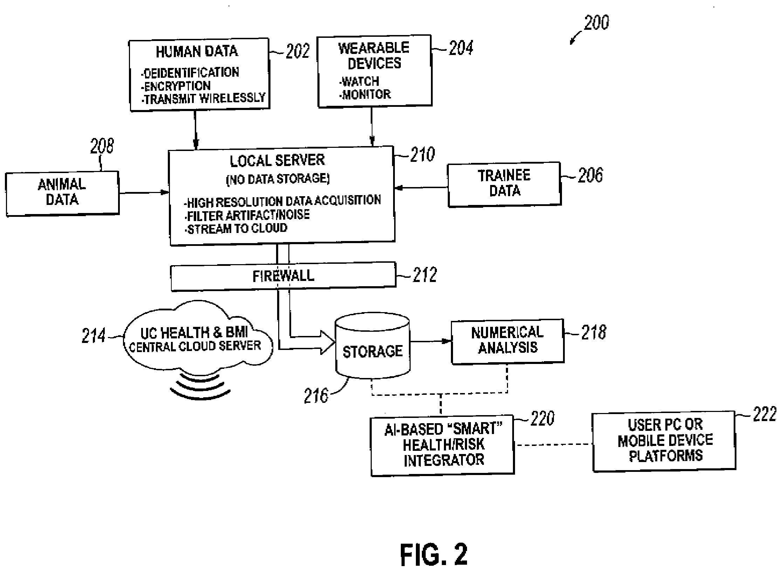

Referring now to Fig. 2, a hardware infrastructure 200 for implementation of one embodiment of the present invention, known as CHAOS can be explained in greater detail.

In this embodiment, critical care data from an individual is gathered via a data collection system 202 as is typically available in an ICU, other hospital environment or other settings. In the setting of a hospital, physiological waveform data from bedside monitors will be automatically de-identified, encrypted (e.g., AES 256-bit HIPAA compliant) and securely transmitted to a central cloud server and linked to clinical data behind the firewall using a global user identifier (GUID) generated via a SHA-2 cryptographic hash function. After waveforms have been collected, analyzed (see below) and integrated (see below), the risk for each patient will be shown via a display like the standard reporting system for that particular environment or application, such as the standard ICU telemetry reporting system.

Additional data may be gathered from non-hospitalized healthy persons, such as persons engaged in training, or ambulatory patients for delivery to the local server and inclusion in the data sets, using wearable devices 204 such as heart-rate / blood pressure monitoring wearable devices, implanted or cutaneous monitoring systems, and the like. In these settings, a portable device comprised of a microprocessor will collect, encrypt and analyze the data and/or securely transmit it to a central cloud server.

In addition, data 206 from healthcare providers such as physicians, nurses, nurse practitioners or medical trainees, etc., personal trainers, or family members of a subjective nature (e.g., patient or caregiver/family perception of patient condition, color, energy, etc.) can be collected by a handheld device or local server for use in assessing healthcare / training quality. Finally, animal data 208 for comparison and assessment may be collected for evaluation and inclusion in data sets to allow extraction of risk factors for animal populations that may be predictive of human physiology.

In one embodiment, the gathered data is delivered via a local server 210 through a firewall 212 to a cloud server 214, where it is stored and indexed for later analyses. Virtual and/or physical servers coupled to the storage 216 of the cloud server may then perform analytic applications 218 operating upon the collected data, as discussed in greater detail below. Those analytic applications comprise collectively an artificial intelligence based health / risk integrator 220 for producing health and risk factor assessments for individual patients or populations, which may be delivered from the cloud server to user PC or mobile device platforms 222.

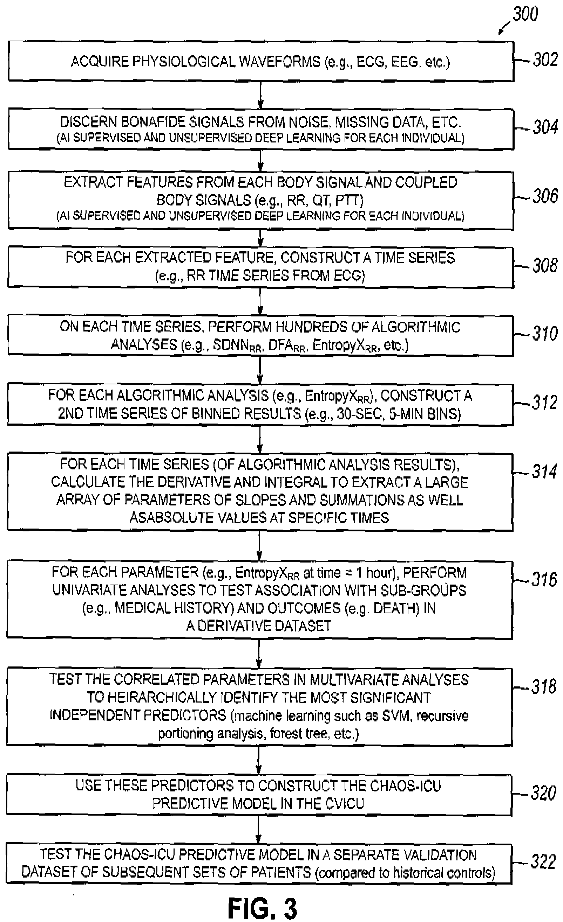

Fig. 3 is a flow chart 300 providing an overview of the process implemented in this system for collection and evaluation of data. This process comprises the collection 302 of physiological waveforms of potentially multiple varieties and types, discernment 304 of bona fide signals from noise and missing data via an AI / deep learning unsupervised process, and extraction 306 of features from each body signal and coupled body signals via a similar method. For each body signal a time series is constructed 308, and for each, a large number (e.g., hundreds) of algorithmic analyses are performed 310 using methods such as EntropyX$_{QT}$ described in the inventor's previous publications, to create 312 a second series of binned results each representing a time period of short scale, e.g., 30 seconds to 5 minutes in length. Next, for each time series the derivative and integral is computed 314 to develop an array of parameters of slopes and summations as well as absolute values at each specific time. Next, the parameters are tested 316 in a multivariate analysis to hierarchically identify the most significant independent predictors using machine learning techniques such as SVM, recursive portioning analysis, forest tree, etc. These predictions then are used to form 320 an individualized predictive model for the subject's health, and/or various conditions and risk factors thereof, which are tested 322 in a separate validation of subsequent patient data sets, providing a continuous supply of automated learning material for the algorithm to continue to refine its reference library.

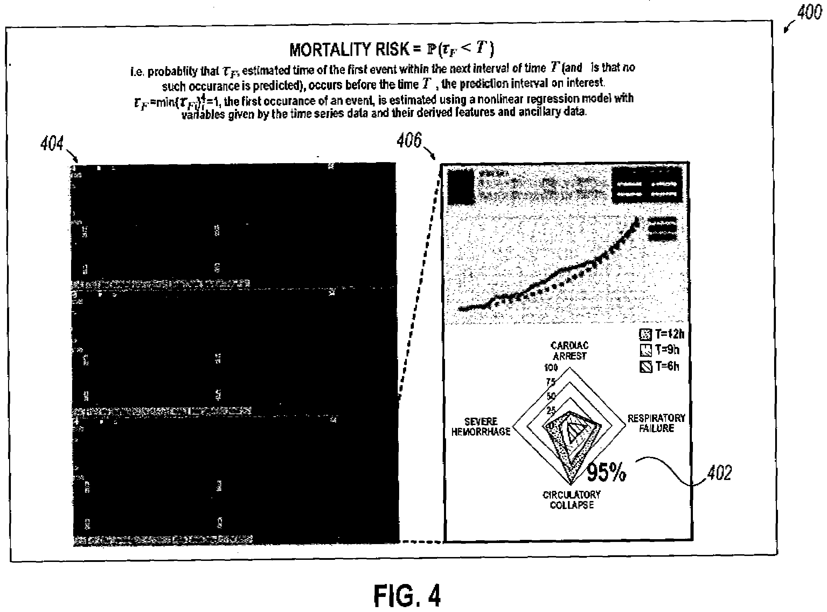

Fig. 4 is an example of a display 400 providing a risk score 402 for a patient which can be developed using the described system. When a high risk score is calculated for a patient, the clinicians taking care of that patient will be notified that the patient has been identified as being at high risk for an impending adverse event. The notified clinicians will be directed to a single stationed display (404) located next to the standard stationed display for ICU monitors. The CHAOS display will look and operate just like the ICU display except that it will indicate either a "High" or "unremarkable" value for the CHAOS score. For patients with a "high" risk display, the patient's clinician will be able to select that section of the screen to access the actual high risk score and its trajectory (406).

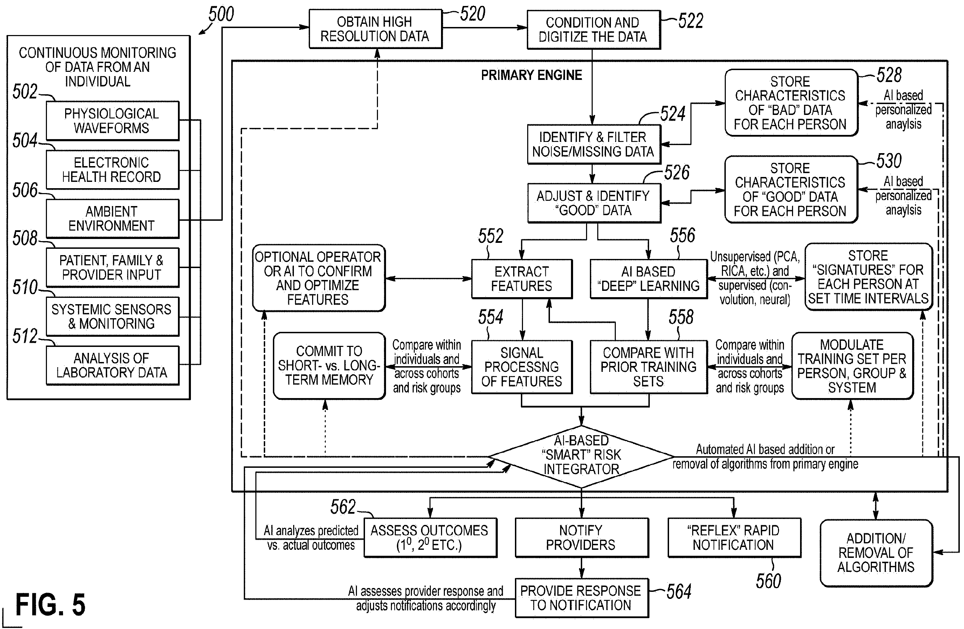

Fig. 5 presents the data flow for the CHAOS system. The CHAOS system is "smart" based on its AI self-optimizing properties. As seen in Fig. 5, numerous data sources 500 including physiological waveforms 502, electronic health record data 504, ambient environment data 506, patient, family or care provider qualitative input 508, systemic sensor and monitor data 510 and analyses of laboratory data 512 are fed 520 at an adequate resolution to the system, where it is conditioned 522 and filtered 524 for noise and missing data and then adjusted to identify 526 "good" data of the original set. The filtering process 524 and identification process 526 utilize learned characteristics 528 of noise or other "bad" data and characteristics 530 of "good" data (i.e., predictive data) for each individual and/or groups of individuals, developed through machine learning processes using the results of prior clinical outcomes and the patient's demographics and medical history information, to improve filtering of noise or data and selectivity of useful data.

In these and subsequent processes, reiterative comparison between prediction and actual clinical outcome and/or adverse or undesired events, through a series of feedback loops permits machine learning in the system, adapting accordingly by adjusting the weight of each independent predictor in the risk integrator for specific individuals and illness groups; including or excluding additional signals and/or predictors; augmenting and improving data analysis; and collecting additional data. These characteristics of the primary analysis engine 550 provide the predictive power of the inventive system.

The self-optimizing properties that are used to iteratively compare the outcomes between predicted and actual over the course of the monitoring period for an individual or a group of individuals allows for a learning component by using the following: (a) adjusting the weights of individual predictors for specific illness and individuals, (b) periodically testing and including or excluding signals and/or predictor(s) as needed to increase the multivariate predictive accuracy; (c) data analysis augmentation and improvement, and (d) collection of more data.

The integrator can also self-replicate and extend its components to include new input sources and analyses. For example, monitoring data (502) are used to assess instability of integrative physiological function based on linear and non-linear dynamic analyses (e.g., EntropyX) of body signals and to calculate the likelihood of an impending, new catastrophic event using established and novel algorithms. Data in an EHR 504 are used to assess traditional risk factors (e.g., diabetes, coronary artery disease), clinical measures (e.g., prophylactic treatments, vital signs, laboratory values), and other risk scores (e.g., APACHE, SAPS, biomarkers) contribute to the integrator's predictive accuracy. Data in 506 includes assessment of ambient environment. Data from 508 compares the patient/family's and providers' categorical impression on whether the patient's condition is improving, worsening, or unchanged. Data from 510 provides an assessment of available resources and personnel in the vicinity of the monitored individual. Finally, data from 512 allows incorporation of laboratory data such as blood plasma count, lipids, etc. and/or new measures of laboratory data such as but not limited to the entropy rate of fractionated serum lipoprotein. The personalized risk score will be calculated for each individual by integrating the data from 502-512 and construction of predictive models to predict outcomes using our novel AI-based approach.

During the training process, a cross-validation technique is used to learn model parameters, e.g., a leave-one-out strategy in which all of the patients in a cohort are used as a training set to evaluate the predictive capacity on the held-out patient. To obtain individualized prospective data, this process is repeated for each person. The optimal predictors are identified to minimize overfitting and achieve the highest possible predictive accuracy while utilizing cross-validation technique. To identify 552 useful features for prediction, pre-processing 554 (e.g., standardization, normalization, reclassification), an iterative process of feature engineering, feature selection, model generation and model-feature assessment is implemented. Predictors that contain missing values are excluded. After the identification of key features, k-means and hierarchical agglomerative clustering methods 556 are used to find similar groups of data to clinically identifiable states (or other novel states to merit further study). Feed-forward neural networks are trained to assign 558 individual data-points to the classes where well-defined clinically relevant classes of diagnoses or outcomes are available (e.g., morbidity, readmission likelihood). The advantage of using neural networks is that sophisticated multi-modal classification using convolutional layers and time-series data can be combined directly with static features within the same classifier. Prediction can also be done by taking future states as the classes. In addition, for predicting the future course of the time-series, time-series data can be used directly using recurrent neural networks. Machine learning techniques can also be used to visualize high-dimensional data in 2-D and 3-D spaces.

Outputs generated by CHAOS can include real time "reflex" or conventional notifications 560 to providers, or assessments 562 of predicted outcomes for comparison to actual results (for feedback to later refinement) and collection 564 of care provider / trainer responses to notifications, also for feedback to later refinement.

As will be appreciated, CHAOS comprises a diagnostic system, method, apparatus, computer algorithm and display with therapeutic intent initially in the cardiovascular field but also relevant to diverse fields such as sleep disorders, respiratory failure, hemorrhage, shock and neurologic impairments, athletic training and assessment, education and training of health care trainees, and caregiver assessment. The theoretical basis is novel, rooted in fundamental science, a priori hypothesis-driven and well supported by recent investigations. In the physical sciences, turbulent flows give rise to some of the most complex signals, e.g., ocean waves, waterfalls. These manifest as spatio-temporal arrays, posing some of the most daunting challenges in physics and mathematics. Surprisingly, this class of dynamics seems connected to multiscale functional properties essential for life itself.

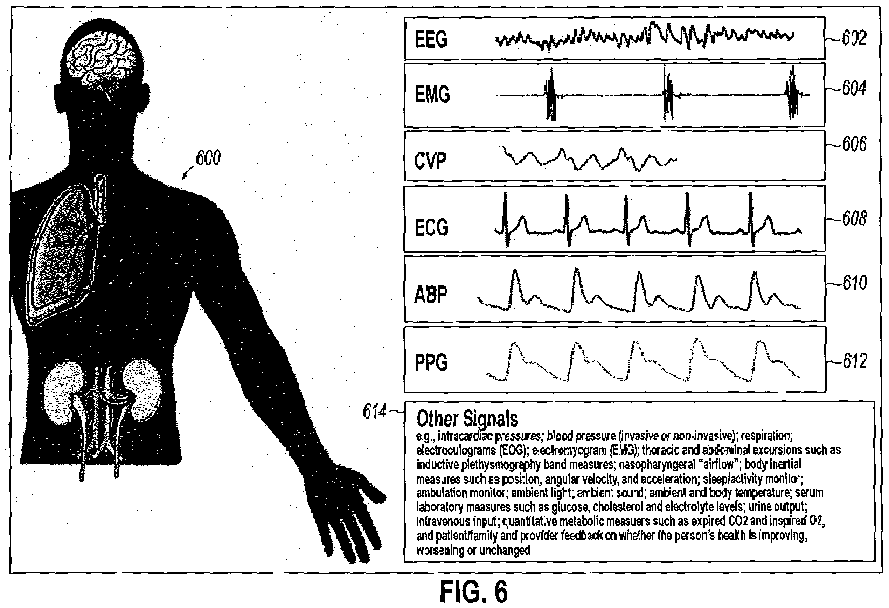

Referring now to Fig. 6, the invention may be applied to a wide variety of physiological waveform signals from an individual 600, including but not limited to brain and nerve electrical activity from electroencephalogram EEG 602, electromyogram (EMG) 604, central venous pressure readings CVP 606, cardiac electrical activity from electrocardiograph (ECG) 608, ambulatory blood pressure ABP 610, pulse photoplethysmography (PPG) 612, and numerous other signals 614, such as other invasive and non-invasive blood pressures, intracardiac pressures, heart rates or other measures, and other waveform signaling intracardiac pressures, blood pressures (invasive or non-invasive), respiration measures, electrooculograms (EOG), thoracic and abdominal excursions such as inductive plethysmography band measures, nasopharyngeal "airflow" measures, body inertial measures such as position, angular velocity, and acceleration, data from sleep/activity monitors, ambulation monitors, ambient light measures, ambient sound measures, ambient and body temperature measures, serum laboratory measures such as glucose, cholesterol and electrolyte levels, urine output, intravenous input, quantitative metabolic measures such as expired $CO_2$ and inspired $O_2$, and patient/family and provider feedback on whether the person's health is improving, worsening or unchanged.

Turning now to a more detailed explanation of one implementation of the present inventive concepts, in one embodiment, at set intervals, e.g., 15 minutes, the present invention will calculate non-linear and conventional linear (deterministic) statistical parameters for all available physiological time series data. A limitation of conventional parameters is that a monotonic relationship is assumed between measured values and risk, e.g., because both bradycardia and tachycardia may be associated with adverse events, the present invention can represent heart rate by its P value in the overall distribution of observed heart rates, or map risks to polynomials. Further, pre-specified candidate predictors will be modeled with restricted cubic spline transformations to reveal non-monotonic and non-linear associations.

The present invention will calculate parameters of joint measures from physiological signals (e.g., RR with QT intervals, RR intervals with respiratory rate, etc.) using cross correlation, mutual information, and cross entropy analyses. For example, a positive correlation coefficient would be expected between RR intervals and respiratory rate in healthy individuals because respiratory sinus heart rate fluctuation or variation due to circadian oscillation are not necessarily markers of disease. However, physiological systems are highly coupled and often behave non-linearly, especially during illness, and as such, subtle subclinical physiological changes indicative of critical illness may not be discerned by deterministic measures. In this regard, the non-linear approaches will complement conventional deterministic measures.

Because parameters will be calculated for each time epoch of predictive monitoring (ranging from minutes to hours and days), there will be many inputs per patient and events. The statistical significance of the model coefficients will be adjusted for repeated measures using the Huber-White method to modify the variance-covariance matrix from the maximum likelihood logistic regression fit. This approach will correct both for unequal variances and correlated responses from individual patients.

The predictive models include standard clinical measures (e.g., lab values) and traditional risk factors and risk scores (e.g., APACHE (Knaus, W.A., et al. The APACHE III prognostic system: Risk prediction of hospital mortality for critically ill hospitalized adults. Chest 100, 1619-1636 (1991)), SAPS (Le Gall, J.R., Lemeshow, S. & Saulnier, F. A new Simplified Acute Physiology Score (SAPS II) based on a European/North American multicenter study. JAMA 270, 2957-2963 (1993))) and use multivariate regression and nearest-neighbor approaches to explore combined predictive values (e.g., do markers of inflammation add to EntropyX in dynamic predictive monitoring?). Binary logistic regression models labeling measures from pre-specified intervals, e.g., every 8 hours, prior to event as outcome = 1, and the rest will be marked as outcome = 0. For each patient, the probability estimate will be divided by the incidence to show the relative risk, or fold-increase in risk of an event in the next day. Predictive accuracy will be quantified using a concordance index (c-statistic) and calibration will be assessed using a nonparametric calibration curve comparing predicted and observed probabilities of the event within the pre-specified time window, e.g., 8 hours, for the original and bootstrapped bias-corrected models. This model will be validated internally using bootstrap resampling to estimate the likely performance on a new sample of observations from the same patient population as well as externally in an analogous patient population in another ICU. Success will be defined by hazard ratios, ROC curve areas, net reclassification improvement, and significant P values.

The relationship between two or more distinct physiological time series that are simultaneously acquired from the same individual will be analyzed using multiple univariate and cross-measures including but not limited to the domains of time (e.g., autocorrelation, cross-correlation and covariance), frequency (e.g., Fourier transform, Lomb periodogram, cross-spectra, coherence, transfer functions), wavelet (e.g., cross- wavelet transform), non-linear (e.g., cross-entropy), phase (e.g., Hilbert transform), information (Granger causality and mutual information), and other mathematical and statistical domains using traditional or novel analyses, transforms, estimators or other mathematical calculations.

In the non-linear domain, cross-measures of two or more simultaneous time series in specified time lags and frequency bands will provide important insight in physiological, pathophysiological, clinical and therapeutic processes. Complex processes can operate with a phase shift that manifests as a time lag, e.g., an increase in heart rate may be associated with a decrease in blood pressure with a slight delay. The extent and classes of time lags that separate events in different time series can be specific to physiological, pathophysiological and therapeutic processes. Their specification is empirical and based on large databases of time series collected during health and the early stages of illness.

A detailed description of the use of Electrocardiograph (ECG) signals can illustrate the process for extraction of features from a physiological signal. Similar processes can be applied to other physiological signals with similar effect. For an ECG signal, the feature extraction process works to create length, area and volume time series.

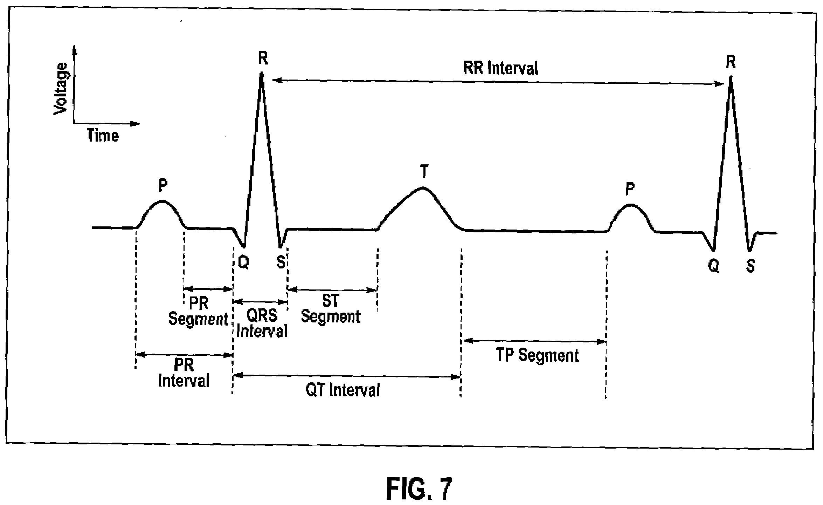

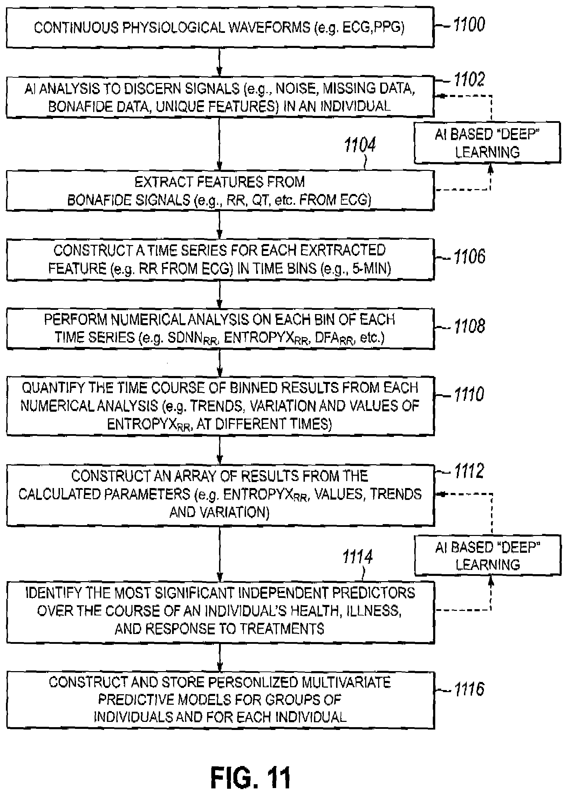

Figs. 7 - 10C are illustrations of a waveform and its processing, and Fig. 11 is a flow chart of steps followed in the processing described herein.

Fig. 7 illustrates the sub-sections of an ECG waveform in order to demonstrate the extraction of features from cardiac electrical signals to determine cardiac electrophysiological dynamics (e.g., activation, repolarization, conduction, impedance, etc). (The significance of various features has already been explored. For example, the dicrotic notch (incisura) reflects vascular tone, i.e., higher arterial tone (lower compliance) can cause the notch to occur earlier and higher on the downward diastolic curve. The upward PPG slope (calculated by second derivative) can reflect the impact of aging or hypertension. The PPG also reflects venous congestion, e.g., larger peaks during diastole. Dynamic ECG+PPG analysis, rooted in fundamental principles of electromechanical coupling, is believed to be unexplored. Pulse transit time (PTT) (time from ECG R-wave to PPG upslope) is linked to hypertension and aging, consistent with the concept that a stiffer vessel conducts a more rapid pulse.)

The extraction of features contemplated herein involves the steps of:

A. Sensing cardiac electrical activity as a voltage time series from surface electrocardiographic (ECG), sub-surface electrical recording leads, intracardiac electrograms (EGM) consisting of unipolar or bipolar leads placed on the endocardium or epicardium connected to an implanted device, or other measures of electrical activity (Fig. 11, Step 1100)

B. Converting the analog values of the ECG voltage time series to digital values at an adequate sampling rate (preferably 1000 Hz or higher)

C. Separating the digitized ECG time series into bins of a specified duration (e.g., 30 sec, 5 mins, 60 mins, 24 hrs)

D. For each bin, extracting features from ECG waveforms for one or more consecutive heartbeats using peak detection, fiducial markers and waveform morphology detection algorithms to extract features (Fig. 11, Steps 1102, 1104) such as periods of atrial activation (PQ, PR), ventricular activation (QRS), repolarization (JT), peak of repolarization (T) to end of repolarization (TpTe), ventricular activation (RR), the entire atrioventricular activation and repolarization (PRQRST), and consecutive ventricular activations (RR) (see Figs. 7-8) as well as machine learning algorithms such as self-organized feature maps (SOFM), restricted Boltzmann machines (RBM), and deep or shallow auto-encoders to extract unique features specific to an individual, group and/or outcome

E. For each extracted feature in a bin, constructing a sequence of intervals including the length (duration), area (e.g., voltage-time integral) and volume (e.g., total sum of the absolute areas from two or more available vectors/leads, resulting in the construction of a length time series, area time series, and volume time series for said feature. (Fig. 11, Step 1106)

F. For each time series of N intervals for each feature in each bin, performing a numerical analysis to quantify the time course of the binned results (Fig. 11, Steps 1108, 1110). In one embodiment, this involves constructing a template and comparing the extracted feature interval for each heartbeat to the template for that bin and consecutive bins as follows:

(1) Given a signal $x(t)$ over the time interval $t \in I$, partition $I$ into $N$ subintervals, $I_n = [t_n, t_n + L_n)$, where $L_n$ is the length of the interval.
(2) Define a template, $\varphi(\tau)$, for $\tau \in [0, \Delta t]$, where $\Delta t$ is the template length.
(3) For the chosen template and for all the subintervals $i = 0, 2, \dots, N - 1$:

(i) Define the error function

$$E_i(\alpha) = \int\limits_0^{\Delta t} \left[ x\left(t_0^{(i)} + \alpha\tau\right) - \varphi_k(\tau) \right]^2 d\tau.$$

This measures the difference between the template and the signal on a time-stretched subinterval.

(4) Define a template, $\varphi(\tau)$, for $\tau \in [0, \Delta t]$, where $\Delta t$ is the template length.
(5) For the chosen template and for all the subintervals $i = 0, 2, \dots, N - 1$:

(i) To determine the time-stretched subinterval length, the error function is minimized and the global minimizer is computed. The optimal time-stretched subinterval length is then $\Delta t_i^* = \alpha_i^* \Delta t$, where $\alpha_i^*$ is the minimizer of $E_i(\alpha)$.

(ii) If the value of the error function at the minimizer, $E_i(\alpha_i^*)$, is above a specified threshold, then the template does not match the time-stretched signal sufficiently well and the template would be rejected.

(iii) This generates a discrete time series of stretched subinterval lengths, $L_i^*$, for $i$ = 1, 2, ..., N.

(6) Minimizer algorithm: To minimize the error function, a modified version of bisection method is used as follows:

(i) Initially set $\alpha$ = 1 and $\Delta\alpha$ = 0.02

(ii) The error function is evaluated at the following values: $\alpha = E(\alpha - \Delta\alpha)$, $b = E(\alpha + \Delta\alpha)$, and $c = E(\alpha)$.

(iii) Modify $\alpha$ or $\Delta\alpha$ based on the following:

1) If $\alpha$ = min($a$, $b$, $c$), then set $\alpha = a$

2) If $b$ = min($a$, $b$, $c$), then set $\alpha = b$

3) If c = min($a$, $b$, $c$), then set $\Delta\alpha = \Delta\alpha/2$

(iv) Iterate steps 3 and 4 until $\Delta\alpha < \varepsilon$, where $\varepsilon$ is a given tolerance, or a maximum number of iterations is reached

(7) If the characteristics (e.g., morphology, significant deviation in value from moving average, etc) for an extracted feature for each heartbeat in a bin do not match the template at or above a specified threshold (e.g., $\geq$85%) for a certain number of consecutive beats and the frequency of failed matches increases towards the end of said bin and continues to increase in the next bin, then a new template will be formed with overlap between the two bins from the point the matches started to fail such that the number of failed matches are minimized and similar in both bins and subsequent bins.

(i) In this manner, the templates are refined with each successive construction, resulting in better processed beat signals and better matching.

(ii) At the same time, the interval waveforms are not significantly improved with further template construction, unless the beat signals that are fed into the computer change their characteristics and that then would result in changing the template waveform.

G. For each time series of N intervals for each feature in each bin, calculating linear-based (e.g., time domain, frequency domain) and nonlinear (e.g., detrended fluctuation analysis, recurrence plot parameters, sample entropy, EntropyX) parameters for each bin (Fig. 11, Step 1112)

(1) further calculating linear and nonlinear parameters from combined analysis of two or more interval time series for each bin

(2) further calculating fluctuation analysis to accurately resolve microcomponent perturbations in signal that are too small to be resolved via direct measurement but that can be extracted using stochastic methods, e.g., ensemble averaging over multiple heart beats over the course of a ramp in voltage, offset subtraction by fitting a straight line to the initial part of the ramp response or mean waveform fitting of a smooth curve over individual heart beats or other forms of nonlinear analysis

H. Forming another feature time series from the statistical moments calculated for each bin over the course of monitoring of an individual.

Through the described process, the most significant independent predictors of an individual's health, illness or response to treatment can be identified (Fig. 11, Step 1114) and then a personalized multivariate predictive model for groups of individuals, and or each individual, can be created and stored (Fig. 11, Step 1116).

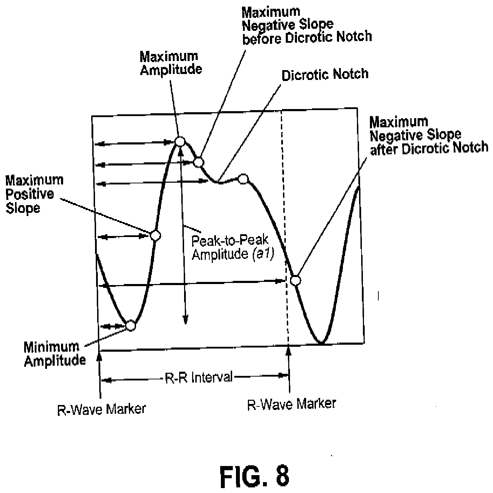

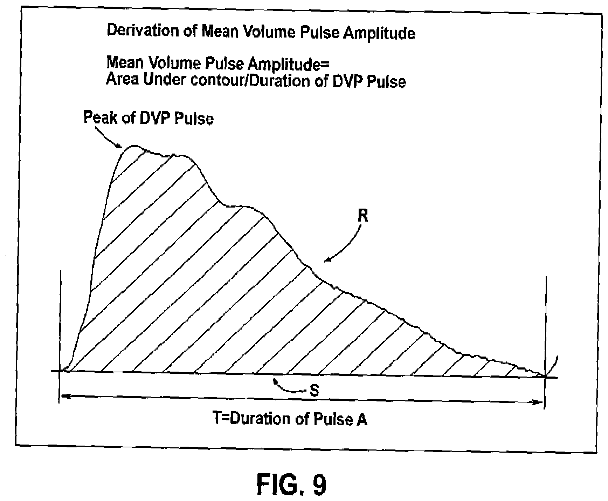

Referring now to Figs. 8 and 9, merely by way of example and not intended to be considered limiting, these figures illustrate how the feature extraction process works to create length, area and volume time series, from PPG waveform signals to determine the dynamics of activation, relaxation and patterned variability in blood flow, volume and pressures, said method comprising sensing PPG as a voltage time series in analog values from changing absorbance of different wavelengths of light or other measures and performing the analysis as outlined for ECG.

The above may be repeated for other physiological and environmental signals, e.g., intracardiac pressures, electroencephalogram (EEG), respiration, saturation of peripheral oxygen or pulse oxygenation signals, blood pressure (invasive or non-invasive), electrooculograms (EOG), electromyogram (EMG), thoracic and abdominal excursions recorded by inductive plethysmography bands, nasopharyngeal "airflow" (e.g., detected by a nasal-oral thermocouple), pulse oximetry, intracardiac electrogram (EGM), body position (e.g., using motion detector, gyroscope sensor, etc), sleep/activity monitor, ambulation monitor, ambient light (e.g., light sensor), ambient sound level, temperature, serum glucose and electrolyte levels, urine output, intravenous input, and patient/family compared to provider feedback on whether the patient appears improved, same, or worsened from prior assessment.

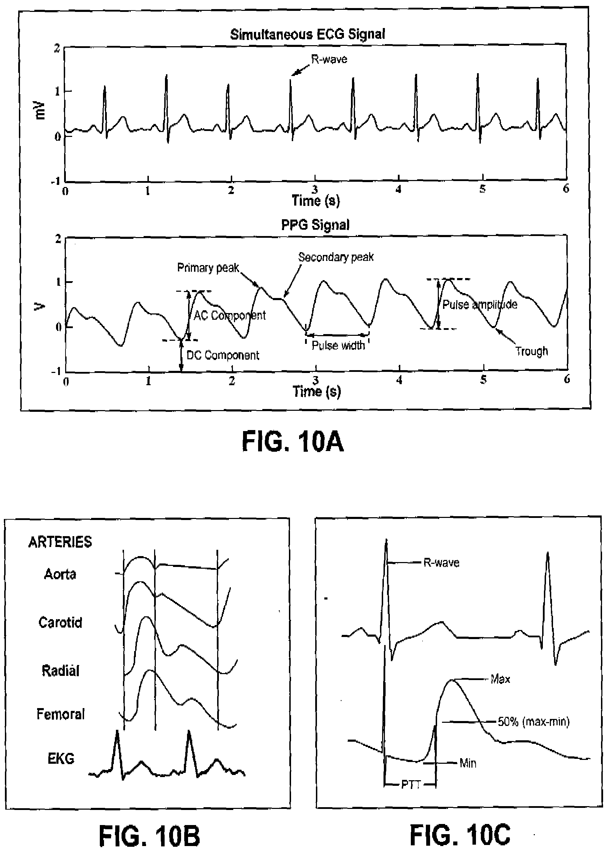

Referring now to Figs. 10A - 10C, the above may be repeated to extract a sequence of N time intervals from two or more distinct simultaneously measured physiological signals (e.g., ECG and PPG as shown in Fig. 10A) and/or the same signal measured at different locations (e.g., PPG at different locations as seen in Fig. 10B), each time interval determined from two consecutive beats using a peak, waveform shape detection and machine learning algorithms, with the start of one interval determined from a certain physiological signal and the end of that interval determined from another distinct signal (e.g., PPT determined from R wave of ECG to max positive slope of onset of PPG as seen in Fig. 10C).

Performing iterative supervised and unsupervised "deep" learning using artificial intelligence and comparing to prior measures in the same individual at an earlier time point as well as to a training set for a relevant group (e.g., gender, race, disease group) as well as from asymptomatic "healthy" individuals with little or no disease.

Reassessing laboratory measures and biomarkers, e.g., nonlinear calculation of the entropy of serum cholesterol particles.

Feeding the results into the "smart" risk integrator that uses artificial intelligence to account for missing data, short-term and long-term memory for deep learning using neural networks, and cerebellar-type reflex for rapid identification to produce acute warnings (refer to schematic of risk integrator).

A. Both the type of data collected and analyses employed are rooted in fundamental mechanisms and modern theories of systems biology. The novel self-optimizing properties to reiteratively compare between predicted and actual outcomes allow for a "learning" component by (1) adjusting weights of independent predictors for specific illnesses and individuals, (2) augmenting and improving data analysis, and (3) collecting more data.

B. In case of the need to augment inputs and expand analysis, the integrator can self-replicate and extend its components to include new input sources and analyses.

C. During training, model parameters will be learned by cross-validation, i.e., a leave-one-out strategy with all patients in the collective reference database as the training set to evaluate the predictive capacity on the held-out patient. This will be repeated per person to obtain individualized prospective data.

D. Optimal predictors to achieve the highest possible predictive accuracy while utilizing cross-validation to minimize overfitting.

E. After pre-processing (e.g., standardization, normalization, reclassification), an iterative process of feature engineering, feature selection, model generation and model/feature assessment will identify useful features for prediction.

F. Once key predictors are identified for each individual and group, methods such as k-means and hierarchical agglomerative clustering will be used to find similar groups of data corresponding to clinically identifiable states (or other novel states that merit further evaluation).

G. In cases where well-defined clinically relevant classes of diagnoses or outcomes are available (e.g., morbidity, readmission), feed-forward neural networks will be trained to assign individual data-points to these classes.

(1) An advantage of using such neural networks is that, by using convolutional layers, time-series data can be combined directly with static features within the same classifier, resulting in sophisticated multi-modal classification.

(2) This can be used for prediction by taking future states as the classes.

H. In addition, time-series data will be used directly for predicting their future course using recurrent neural networks. Machine learning will also be used to visually represent high-dimensional data in 2-D or 3-D spaces.

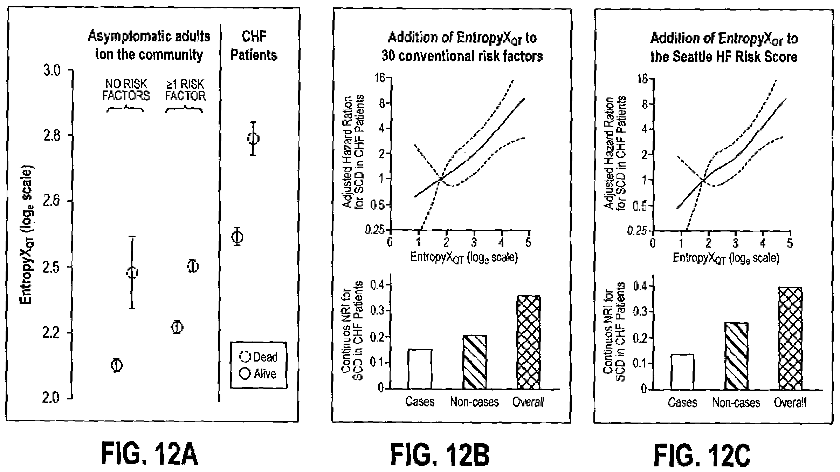

Turning now to Figs. 12A et seq., the use of entropy measurement in accordance with the present invention can be elaborated. In the physical sciences, turbulent flows give rise to some of the most complex signals (e.g., ocean waves, waterfalls). They manifest as spatio-temporal arrays, posing the most daunting challenges in physics and mathematics. Surprisingly, this class of dynamics is linked to the key functional properties essential for life. Building upon this and newer concepts of entropy and machine learning, the inventor herein introduced a powerful new strategy (EntropyX) to accurately measure the information entropy of physiological time series that will illuminate the subclinical pathophysiologic world within us, reported in the above-noted patent applications.

In prior studies, various approaches have attempted to measure mortality risk via non-linear analyses of ECG, including Poincare plots, various forms of entropy analyses and detrended fluctuation analysis (DFA). Notably, in a large randomized clinical trial, the use of sample entropy to reveal subclinical ECG signatures of impending shock led to early diagnosis and therapy, thus reducing mortality. Through new concepts of entropy estimation and machine learning, the inventor has improved upon sample entropy to develop new, more powerful algorithms for measuring the information entropy of not just the ECG but of all time-varying body signals. This set of algorithms is termed EntropyX, providing several advantages over prior strategies for linear-based and non-linear analyses, including sample entropy.

For example, as illustrated in Figs. 12A-12C EntropyX quantifies the repetition of distinct patterns underlying physiological variability to provide unique prognostic insight. As seen in Fig. 12A. $EntropyX_{QT}$ values (mean$\pm$SE) are plotted on a $log_e$ scale for individuals that died (in red) or had no events (blue) over follow up of asymptomatic adults with no risk factors (N=384) and with $\geq$1 risk factor (N=5,667), and of heart failure patients (N=694). Fig. 12B shows curves representing multivariate-adjusted hazard ratios (solid line) with 95% confidence intervals (dotted lines) based on restricted cubic splines with knots at the 5th, 35th, 65th, and 95th percentiles of $EntropyX_{QT}$ distribution. The reference value (hazard ratio = 1) was set at 1.79, the 10th percentile of the $EntropyX_{QT}$ distribution. In the bottom, the net reclassification improvement is plotted for cases (cyan), non-cases (red), and overall (black). Adding $EntropyX_{QT}$ increased both sensitivity and specificity of an extensive set of conventional risk predictors, e.g., improved net reclassification for SCD by >35%. Fig. 12C presents the same data as Fig. 12B except $EntropyX_{QT}$ was added to Seattle Heart Failure risk score.

Unlike conventional measures of variability, EntropyX does not require equally sampled time intervals or extensive preprocessing of data. Unlike prior non-linear analyses (e.g., sample entropy, approximate entropy), EntropyX is insensitive to the degree of tolerance allowed for matching templates and outlying points (e.g., noise, ectopic beats). Unlike approximate entropy, frequency domain and geometric measures such as Poincaré plots, EntropyX produces consistent results in both short and long time series. Because EntropyX can be applied to all body signals, future studies can extend the ECG and PPG studied in this proposal to other body signals (e.g., electroencephalogram) as a potential conduit for continued discovery and refinement of the proposed PSG-based mortality risk prediction model.

In a multicenter prospective clinical study of heart failure patients, the inventor has demonstrated the clinical utility of EntropyX of cardiac repolarization ($EntropyX_{QT}$), one of the EntropyX algorithms. Incorporation of $EntropyX_{QT}$ to a comprehensive set of established predictors strongly and independently improved SCD risk stratification, as illustrated in Figs. 12A-12C. The inventors's preliminary data suggest that the benefit of this novel strategy is even greater in asymptomatic adults with less advanced heart disease, including those ostensibly healthy without any risk factors as well as those considered low-risk with only a few risk factors (Fig. 12A).

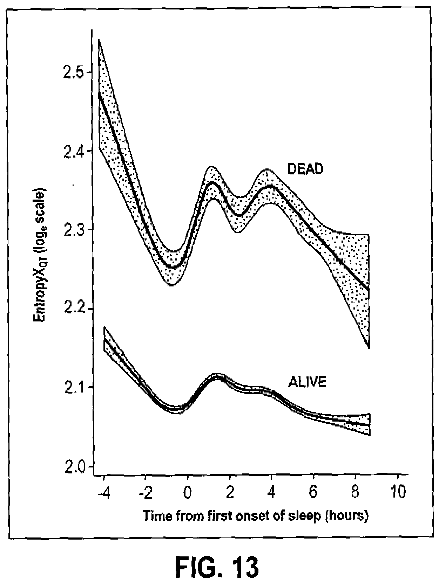

More recent preliminary analyses by the PI of PSG ECG waveforms in the Sleep Heart Health Study (SHHS) suggest the discriminative power of $EntropyX_{QT}$ is further increased at the onset of sleep. This is illustrated in Fig. 13. As seen there, the time course of $EntropyX_{QT}$ ($log_e$ scale) before and after first onset of sleep. Cubic spline plots (black lines) of EntropyXQT with 95% confidence intervals (shaded areas) are shown for those who died in follow-up (N=1,066; in brown) and those without any event (N=5,248; in blue). $EntropyX_{QT}$ values and slopes were markedly different between groups. Association with sleep states (e.g., REM, non-REM) can also be expected in further study. Extending the EntropyX paradigm to PSG-based mortality risk stratification of asymptomatic community adults has potential to yield ground-shifting clinical impact.

The present invention has thus established that electrocardiographic (ECG) and pulse oximetry photoplethysmographic (PPG) signatures during sleep provide unique prognostic insight into mortality risk of asymptomatic community adults. CHAOS-sleep, a PSG-based mortality risk assessment is expected to spark exciting new avenues in collaborative sleep and cardiovascular research.

Analysis of sleep data will proceed by calculating the large array of parameters (potential predictors) from the time course of 5-min binned analyses of each time series per feature extracted from each body signal. For this, the MESA-sleep training set is ideal because these waveforms have been extensively screened and manually annotated.

On each 5-min bin of each time series of an extracted feature, the following analyses will be performed: (1) Conventional ECG analyses will be performed, including power spectra of RR and QT intervals to assess coherence of the spectra over the physiological frequency range. We will generate the cross spectrum using the Blackman-Tukey method and calculate coherence as reported. Heart rate variability (HRV) in the time domain will be calculated as the standard deviation of NN intervals (SDNN) over 5 minutes. The HRV in the frequency domain will be calculated using fast Fourier transform and Lomb-Scargle periodogram with bands of high frequency (HF; 0.15-1.0 Hz) and low frequency (LF; 0.04-0.15 Hz). A range of normal values for QT variability index and HRV in the time and frequency domains have been established. (2) Nonlinear ECG analyses will be performed using EntropyX and DFA. The DFA offers distinct insight by measuring correlations within HRV. The correlations will be divided into short-term ($\alpha_1$, range $4 \leq n \leq 16$) and long-term ($\alpha_2$, $16 \leq n \leq 64$) fluctuations. For example, $0 < \alpha < 0.5$ indicates that a large value is followed by a small value and vice versa; and $0.5 < \alpha < 1.0$ indicates a large value is followed by a large value. An $\alpha$ of 0.5, 1.0, >1.0, or >1.5 indicates white noise, 1/$f$ noise, different types of noise, or brown noise (integral of white noise), respectively. In contrast, EntropyX examines changes in patterns of variability such that the presence of more frequent and more similar patterns lead to lower EntropyX values. EntropyX exploits information in the ordering of the interval time series and quantifies the degree to which patterns of fluctuation repeat themselves. More self-similar fluctuations can imply an increased coupling of periodic processes among organ systems that typically are a signature of health, e.g., respiratory sinus arrhythmia. Based on our preliminary findings, we expect the refined analysis with EntropyX to reveal the subtle instabilities of electromechanical impulse propagation, repolarization/relaxation and coordination in the heart, along with the likelihood that a new, out-of-sync electromechanical signal will occur to trigger SCD or initiate contractile failure.

The time course of the 5-min bin results for each calculated parameter will be quantified by absolute values and slopes around specific time points relative to the first onset of sleep. Our preliminary data suggest that analysis of time series data after alignment by the first onset of sleep in each individual is critical for revealing important associations with mortality risk. To our knowledge, prior studies have not analyzed PSG time series data in this manner. The resulting large array of parameters will be correlated with sub-groups, including demographics (e.g., age, race, gender), risk factors (e.g., diabetes, body mass index, blood pressure), medications, lab values, serum inflammatory markers, sleep states (awake, REM, non-REM) and sleep characteristics (e.g., apnea, central arousals, time of night, duration of sleep).

The described algorithms for ECG processing may also be adapted to the goal of eliminating the human interaction component of PPG analyses without compromising precision or accuracy. As with the ECGs, time and frequency domain analyses may be performed on the PPG waveforms. In the time domain, the key features include amplitude (related to pulse pressure, stroke volume, vascular compliance), area, width (at the base and at 50% height), the maximum slope (related to $\delta P/\delta T$) and minimum slope (related to speed of vessel relaxation and blood flow). In the frequency domain (e.g., Fourier transformation), the PPG waveforms can be described as the sum of simpler underlying harmonics (sine and cosine waves). The advantage of this approach is that the PPG waveform is primarily composed of three physiologic metronomes, i.e., heart, lungs and autonomic nervous system. The preload information from venous modulation (a component known as DC divided by respiratory modulation) is related to stroke volume variation present in the cardiac portion (a component known as AC divided by stroke volume modulation), providing critical non-invasive insight into the Frank-Starling curve of each individual. Like the ECG, the time course of each calculated parameter per 5-min bin will be numerically quantified at discrete time points in the periods before and after the first onset of sleep. The large resulting array of PPG values and slopes at discrete time points will be correlated with sub-groups.

Once this data is available, then the most important independent predictors may be identified in the tens of thousands of parameters extracted from the time series of features of PPG, and ECG+PPG waveforms. Whereas conventional multivariate statistics preclude analysis of too many covariates (e.g., collinearity), hierarchical analysis using AI is well suited for this. Predictive models will be derived using a combination of conventional statistical (e.g., penalized logistic regression, proportional hazards) and progressive AI_(e.g., support vector machine (SVM), random forests (RF), classification and regression tree (CART), and supervised and unsupervised deep learning techniques. Combinations may be explored through forest trees to consistently identify the most important independent predictors.

Using the data generated, a PSG-based mortality risk score (CHAOS-sleep) may be developed, specific to subgroups (e.g., age, sex, race, history). A risk integration index using proportional hazards models can estimate associations of each candidate predictor with outcomes. All of the key candidates will be assessed individually in models adjusted for subgroups and established predictors. Covariates with p-value <0.05 in initial models will be included simultaneously and only predictors that remain statistically significant will be incorporated into each final individual risk index. Continuous variables will be categorized into tertiles according to their distribution. To generate a simple integer-based point score for each predictor, scores are assigned by dividing $\beta$-coefficients by the absolute value of the smallest coefficient in the model and rounding up to the nearest integer. The final risk integration index per person will be calculated by adding the point scores for all individual independent predictors. An optimal-performing risk index (CHAOS-sleep, Framingham risk score, or a multivariate model comprised of established risk predictors) will be determined by calculating risk discrimination and continuous net reclassification to assess improvement in predictive ability when adding these new risk indexes to conventional factors and predictors (e.g., age, gender, race, diabetes, coronary artery disease, renal function, sleep apnea, etc.). Model discrimination will be assessed by Harrell's C-index. An adjusted hazards ratio and measures of reclassification of 5- and 10- year risks can also be calculated to quantify the extent to which individuals are more appropriately classified into risk categories using CHAOS-sleep vs. model of established risk factors vs. the Framingham risk score. Reclassification tables can be constructed to examine movement of participants between three predicted 5- and 10- year risk categories [e.g., low (<5%); intermediate (5-10%); high ($\geq$10%)] when adding new risk indexes to established risk factors. In addition, model calibration will be assessed by visual inspection of observed vs. predicted risk and modified Hosmer-Lemeshow statistic.

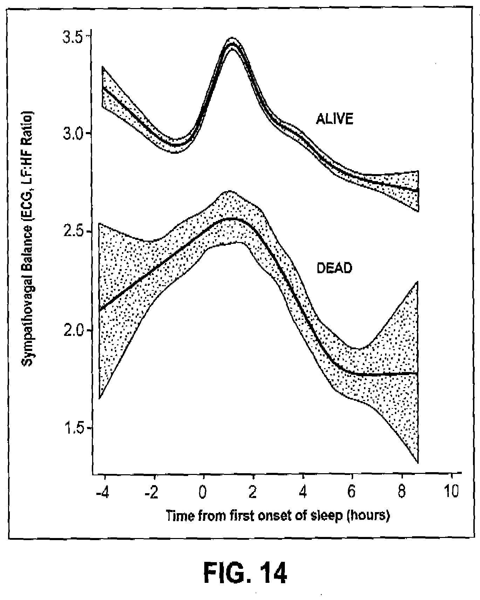

Fig. 14 illustrates the time course of sympathovagal changes before and after first onset of sleep. Cubic spline plots (black lines) with 95% confidence intervals (shaded areas) for the low to high frequency ratio (LF:HF) of ECG's power spectral density (PSD) analyzed by 5-min bins are shown for those who died over follow-up (N=1,066; in brown) and those without any events (N=5,248; in blue). The sympathovagal tone was much lower, along with a blunted sympathovagal response to the onset of sleep in those that died over follow up.

As shown, EntropyX$_{QT}$ has proven important for SCD risk stratification in heart failure patients and predicted SCD in preliminary results in sleep study database. Even conventional HRV measures seem to have greater predictive value, especially at the onset of sleep in our preliminary data. These exciting new preliminary findings indicate the proposed studies are likely to contribute fundamental new mechanistic and prognostic insight. In particular, REM sleep is linked to autonomic stress and may be better examined. Thus, the inventor expects these initial milestone findings to evolve beyond the results presented here to open exciting new avenues in collaborative sleep and cardiovascular research.

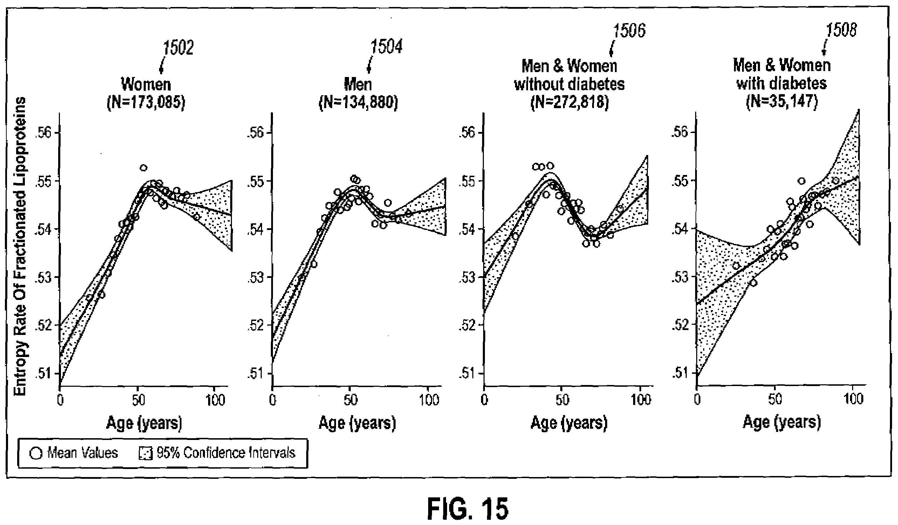

As a second example of the efficacy of the principles disclosed herein, Fig. 15 is an illustration of EntropyXQT calculations applied to fractionated lipoprotein measurements in a population of men and women, illustrating variance between those persons with and without diabetic symptoms. A data series was formed from each individual's lipid-density profile (graphing frequency vs. density from a blood sample); EntropyX$_{QT}$ measures were then computed on these series and the resulting entropy measure graphed vs. subject age for men 1502 and women 1504. While the series are different for each gender, each demonstrates a monotonic increase until approximately age 50, indicative of greater entropy in lipid density concentrations with increasing age. The reductions after age 50 are potentially indicative of selective mortality of patients showing the greatest entropy in lipid density concentrations. Further analysis has revealed a clear correlation of the entropy of lipid density concentration and the absence 1506 and presence 1508 of diabetes in men and women.

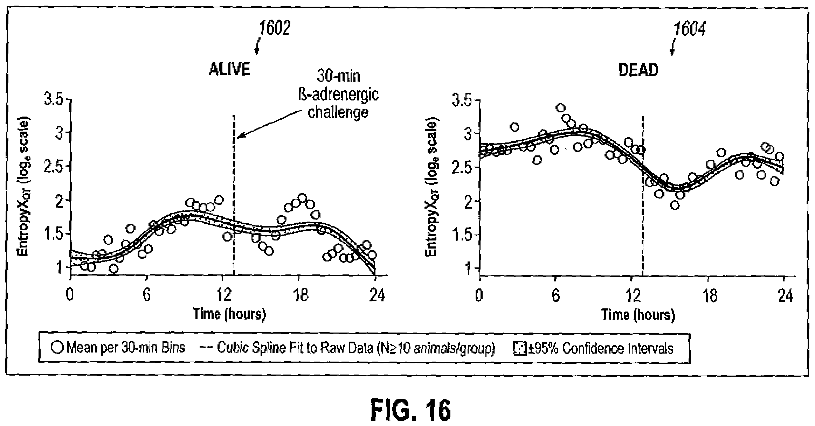

Fig. 16 is an illustration of the efficacy of EntropyX$_{QT}$ ECG measurements on animal populations, showing similar results as in human studies. The data illustrated is from ECG waveforms of guinea pigs, separated into those subjects which survived 1602 and those which deceased 1604 during the experimental period. Again, the EntropyX$_{QT}$ measurement on ECG data demonstrates predictivity of animal mortality risk.

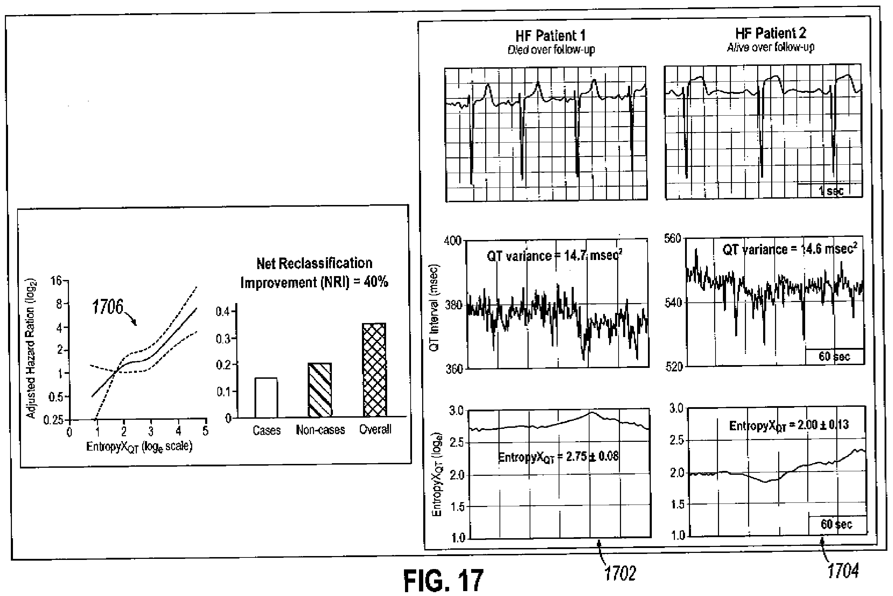

Fig. 17 is an illustration of data produced in a multicenter prospective observational study of 851 heart failure patients showing EntropyX$_{QT}$ measurements taken of ventricular repolarization waveforms. The data show HF patients 1702 who died over follow up show a substantially greater entropy in this measure than those which survived 1074. A regression of hazard ratio vs. EntropyX$_{QT}$ 1706 shows a strong positive and nearly linear correlation indicating prediction of SCD and mortality from this measure over the subsequent 21-69 months.

The invention is not limited to applications at a macrophysiological level. For example, signals may be gathered at cellular level such as to evaluate heart muscle response to electrical stimulation (time from signal to contraction), and this data processed as described above for predictivity of heath, athletic performance, illness or the like.

Use cases for predictive systems according to the invention extend well outside hospitalization. For example, critical waveform data gathered in an ambulance can be processed according to the invention, and the invention may be used in triage such as in a trauma center or emergency room, or in other first responder circumstances. Furthermore, analyses may be applied to triage patients in each step in inpatient and outpatient care, from intensive care units to hospital rooms, to rehabilitations facilities and then to home care. Home care and rehabilitation facilities may apply the invention to determining whether patients are improving or may need return to a higher level of care to avoid additional onset of illness.

The present invention is also applicable beyond the provision of treatment to individuals or prediction of non-emergent illness. Specifically, the present invention provides systems-based analytics for eliminating medical errors, preventable harm and missed diagnoses, thus achieving better clinical outcomes and reducing costs. As is known, healthcare services include numerous potential hazards and can cause preventable complications such as hospital-acquired infections, patient falls, missed billable diagnoses (e.g., paroxysmal atrial fibrillation), failure to follow evidence-based practices (e.g., medication reconciliation, prophylaxis), and poor communication among providers and patients.

The present invention may be applied to enhance the extraction, representation and sharing of health data, patient preference elicitation and personalization of "generic" therapy plans, as well as assess adaptation of providers to care environments and available health expertise, and analysis of patterns in medication reconciliation based on comorbidities, providers and drug classes (including, e.g., appropriate use of antibiotics). The invention, via integration of medical information from the outputs of multiple machines and technologies that monitor a patient's progress, provide evaluation of caregiver performance, as well as better accessibility of patients to medical information to improve the quality of care.

Novel AI-based algorithms of the type described above may be leveraged to identify the sources of medical errors in order to systematically eliminate them and enhance clinical outcomes, deliver patient-centered care, improve patient/family participation, and foster a culture of collaboration, accountability and organizational learning.

Analytics for enhancing training facilitated by the invention include the gathering of trainee performance data via virtual personal assistants, to permit identification of impending exhaustion/failure of trainees. Leveraging innovative "deep" neural network algorithms for analyzing large datasets of performance evaluation patterns and outcomes of medical and allied health professional trainees, the invention may identify trainees that are excelling and importantly, those that may suffer setbacks and would benefit from closer monitoring and early interventi on/gui dance.

To advance public health education requires applying science into clinical practice at superhuman speed, accuracy and scale. The "big data" collected through the invention will serve as an important resource for creating and expanding training programs (e.g., T32, graduate curriculum), providing career development opportunities, generating research studies, securing external funding and facilitating development of new strategies for improving patient outcomes and efficient allocation of healthcare resources. Further, the data gathered will create an open-source, open-platform informatics and analytics infrastructure freely available to the broader academic research community for analyzing human biosignals before/during critical illness to gain rapid and unique insights where simulations alone cannot fully predict the real world and translating data science into personalized medicine.

As noted above, the invention is also applicable in facilitating the development and training of athletes and others for optimal exercise tolerance and performance. By tracking the results of exercise, nutrition and training regimens at a deep level not now available, the invention facilitates the development of new AI-based methods for unraveling unique mechanistic and performance insights that otherwise are not available from conventional approaches. For example duration of activity or exercise or physiological signs during exercise may be used as input signals along with quantitative measures such as strength, sprint times, muscle twitch speed, along with qualitative health measures such as self-assessed or trainer-assessed fatigue and performance assessments, to evaluate training regimens and model ideal regimens for a particular individual or population.

**Claims**

1. A computer-implemented method (300) for assessing the likelihood of an individual experiencing a condition, the method (300) comprising:

    a. providing (302) one or more time-varying signals from continuous monitoring of the individual or the individual's environment, the one or more time-varying signals including one or more physiological signals;
    b. processing (306) the signals to develop signatures for those signals, the signatures being determined based on linear and non-linear characteristics of the signals, phase and time lags of the signals, temporal window lengths of the signals, degree of coupling between different physiological signals;
    c. calculating (320) an integrated likelihood of experiencing the condition based on the developed signatures;
    d. providing the integrated likelihood and/or signatures to the individual and/or a training or health-care provider for the individual; and
    e. updating the displayed integrated likelihood and/or signatures on a time schedule.

2. The method (300) according to claim 1, wherein calculating an integrated likelihood comprises non-linear dynamic analysis of a nonlinear signal derived from at least one of the time-varying signals following discernment of bona fide signals from noise and missing data via an AI process.

3. The method (300) according to claim 1, wherein the time-varying signals comprise continuous time-varying physiological and/or environmental signals, and wherein the signatures are telemetrically derived waveform data.

4. The method (300) according to claim 3, wherein the signals comprise continuous waveform output from one or more of: electrocardiogram (ECG), electroencephalogram (EEG), electrooculogram (EOG), electromyogram (EMG), thoracic and/or abdominal excursions recorded by inductive plethysmography bands; recordings of nasopharyngeal airflow, pulse oximetry, intracardiac pressure, intracardiac electrogram (EGM), temperature, gauge and/or camera-sensed body position, sleep/activity monitor, acid/base monitoring, respiration, acceleration or ambulation monitor, ambient light exposure monitor, ambient sound exposure monitor, ambient temperature monitor, invasive and/or noninvasive blood pressure readings, glucose level monitor, hemoglobin oxygen saturation monitor, arterial blood oxygen and carbon dioxide partial pressure monitor, tissue hemoglobin oxygen saturation monitor, nasopharyngeal airflow, and metabolic activity monitor based on one or more of expired $CO_2$, inspired $O_2$, respiration rate, respiration volume, and laboratory blood measurements including not limited to plasma count and lipid density concentration measurements.

**5.** The method (300) according to claim 1 wherein at least one of the signals associated with the condition further comprises individual, family, and/or health care provider subjective reporting of a relative status of the individual's condition.

**6.** The method (300) according to claim 1, wherein the one or more signals comprise at least one subclinical signature.

**7.** The method (300) according to claim 1, wherein the individual is asymptomatic for the condition.

**8.** The method (300) according to claim 1, wherein the one or more signatures are derived from a population of individuals evidencing the condition or from the individual or both.

**9.** The method (300) according to claim 1, wherein the signals represent continuous monitoring over a time frame of one hour to several days, and wherein the integrated likelihood and signatures are updated on a time schedule is every 1 to 30 minutes.

**10.** The method (300) according to claim 1, further comprising creating a profile of the individual comprising a composite of the individual's time-varying signatures and integrated likelihood of groups of individuals with similar time-varying signatures, storing the signature profile, and utilizing the stored signature profile upon the individual's subsequent treatment or training.

**11.** A system (200) for assessing the likelihood of an individual experiencing a condition in accordance with any one of the preceding method claims, the system (200) comprising:

> a. a data collection means (202) configured to provide one or more time-varying signals from continuous monitoring of the individual or the individual's environment, the one or more time-varying signals including one or more physiological signals;
> b. a central server (210) configured to receive the one or more time-varying signals;
> c. one or more servers (214) coupled to the central server (210) and configured to:

>> process the one or more time-varying signals to develop signatures for those signals, the signatures being determined based on linear and non-linear characteristics of the signals, phase and time lags of the signals, temporal window lengths of the signals, degree of coupling between different physiological signals, and calculate the integrated likelihood of experiencing the condition based on the developed signatures;

> wherein the central server (210) is further configured to provide the integrated likelihood and/or signatures to the individual and/or training or healthcare provider for the individual, and update the displayed integrated likelihood and/or signatures on a time schedule.

**12.** The method (300) according to claim 8, further comprising:

> a. collecting time-varying physiological signals for each individual of the population, wherein the population includes individuals that have experienced a predictive event or condition and individuals who have not experienced the predictive event or condition;
> b. processing the time-varying physiological signals to produce signature profiles for each individual;
> c. comparing signature profiles for those individuals who have experienced the predictive event or condition to signature profiles for those individuals who have not experienced the predictive event or condition to identify a correlative pattern distinguishing signature profiles of individuals who have experienced the event or condition and individuals who have not experienced the event or condition;
> d. computing the integrated likelihood of the event or condition for a candidate by collecting time-varying physical signals for the candidate, processing the time-varying physiological signals to produce signature profiles for the candidate, and identifying whether the correlative pattern appears in the signature profiles for the candidate.

**13.** The method (300) according to claim 12, wherein the event or condition is one or more of mortality or emergence of symptoms of disease.

**14.** The method (300) of claim 13 wherein the correlative pattern comprises one or more of signal variation during sleep onset or a sleep state transition, use or non-use of a drug or pharmaceutical, exposure or non-exposure to an environmental condition or agent.

**15.** The method (300) of claim 13 wherein the time-varying signals comprise one or more telemetrically monitored non-digitized time-varying signatures.

**Patentansprüche**

**1.** Computerimplementiertes Verfahren (300) zum Beurteilen der Wahrscheinlichkeit, dass eine Person einen Zustand erfährt, wobei das Verfahren (300) Folgendes beinhaltet:

    a. Bereitstellen (302) eines oder mehrerer zeitveränderlicher Signale aus der kontinuierlichen Überwachung der Person oder der Umgebung der Person, wobei die ein oder mehreren zeitveränderlichen Signale ein oder mehrere physiologische Signale umfassen;

    b. Verarbeiten (306) der Signale zum Entwickeln von Signaturen für diese Signale, wobei die Signaturen auf der Basis von linearen und nichtlinearen Charakteristiken der Signale, Phasen- und Zeitverzögerungen der Signale, Zeitfensterlängen der Signale und dem Grad der Kopplung zwischen verschiedenen physiologischen Signalen bestimmt werden;

    c. Berechnen (320) einer integrierten Wahrscheinlichkeit des Erfahrens des Zustands auf der Basis der entwickelten Signaturen;

    d. Bereitstellen der integrierten Wahrscheinlichkeit und/oder der Signaturen für die Person und/oder einen Trainings- oder Gesundheitsdienstleister für die Person; und

    e. Aktualisieren der angezeigten integrierten Wahrscheinlichkeit und/oder Signaturen nach einem Zeitplan.

**2.** Verfahren (300) nach Anspruch 1, wobei das Berechnen einer integrierten Wahrscheinlichkeit eine nichtlineare dynamische Analyse eines aus mindestens einem der zeitveränderlichen Signale abgeleiteten nichtlinearen Signals nach dem Unterscheiden von Bona-Fide-Signalen von Rauschen und fehlenden Daten über einen KI-Prozess beinhaltet.

**3.** Verfahren (300) nach Anspruch 1, wobei die zeitveränderlichen Signale kontinuierliche zeitveränderliche physiologische und/oder Umgebungssignale umfassen, und wobei die Signaturen telemetrisch abgeleitete Wellenformdaten sind.

**4.** Verfahren (300) nach Anspruch 3, wobei die Signale einen kontinuierlichen Wellenformausgang von einem oder mehreren umfassen von: Elektrokardiogramm (EKG), Elektroenzephalogramm (EEG), Elektrookulogramm (EOG), Elektromyogramm (EMG), durch induktive Plethysmographiebänder aufgezeichneten Thorax- und/oder Abdominalauslenkungen; Aufzeichnungen von nasopharyngealem Luftstrom, Pulsoximetrie, intrakardialem Druck, intrakardialem Elektrogramm (EGM), Temperatur, Messgerät- und/oder Kamera-erfasster Körperposition, Schlaf-/Aktivitätsmonitor, Säure/Basen-Überwachung, Atmungs-, Beschleunigungs- oder Umhergehüberwachung, Umgebungslichtüberwachung, Umgebungslärmüberwachung, Umgebungstemperaturüberwachung, invasiver und/oder nichtinvasiver Blutdruckmessung, Glukosespiegelüberwachung, Überwachung der Hämoglobin-Sauerstoffsättigung, Überwachung des Sauerstoff- und Kohlendioxidpartialdrucks im arteriellen Blut, Überwachung der Hämoglobin-Sauerstoffsättigung im Gewebe, Überwachung des nasopharyngealen Luftstroms und Überwachung der Stoffwechselaktivität auf der Basis von einem oder mehreren von ausgeatmetem $CO_2$, eingeatmetem $O_2$, Atemfrequenz, Atemvolumen und Laborblutmessungen, einschließlich, aber nicht beschränkt, auf Plasmazahl- und Lipiddichtemessungen.

**5.** Verfahren (300) nach Anspruch 1, wobei mindestens eines der mit dem Zustand assoziierten Signale ferner subjektive Berichte der Person, der Familie und/oder des Gesundheitsdienstleisters über einen relativen Status des Zustands der Person umfasst.

**6.** Verfahren (300) nach Anspruch 1, wobei die ein oder mehreren Signale mindestens eine subklinische Signatur umfassen.

**7.** Verfahren (300) nach Anspruch 1, wobei die Person für den Zustand asymptomatisch ist.

**8.** Verfahren (300) nach Anspruch 1, wobei die ein oder mehreren Signaturen von einer Population von den Zustand aufweisenden Personen und/oder der Person abgeleitet sind.

**9.** Verfahren (300) nach Anspruch 1, wobei die Signale eine kontinuierliche Überwachung über einen Zeitrahmen von einer Stunde bis zu mehreren Tagen darstellen, und wobei die integrierte Wahrscheinlichkeit und die Signaturen nach einem Zeitplan alle 1 bis 30 Minuten aktualisiert werden.

10. Verfahren (300) nach Anspruch 1, das ferner das Erzeugen eines Profils der Person, das eine Kombination aus den zeitveränderlichen Signaturen der Person und der integrierten Wahrscheinlichkeit von Gruppen von Personen mit ähnlichen zeitveränderlichen Signaturen umfasst, das Speichern des Signaturprofils und das Verwenden des gespeicherten Signaturprofils bei der/dem nachfolgenden Behandlung oder Training der Person beinhaltet.

11. System (200) zum Beurteilen der Wahrscheinlichkeit, dass eine Person einen Zustand nach einem der vorherigen Verfahrensansprüche erfährt, wobei das System (200) Folgendes umfasst:

a. ein Datensammelmittel (202), konfiguriert zum Bereitstellen eines oder mehrerer zeitveränderlicher Signale aus der kontinuierlichen Überwachung der Person oder der Umgebung der Person, wobei die ein oder mehreren zeitveränderlichen Signale ein oder mehrere physiologische Signale umfassen;
b. einen zentralen Server (210), konfiguriert zum Empfangen der ein oder mehreren zeitveränderlichen Signale;
c. einen oder mehrere Server (214), die mit dem zentralen Server (210) gekoppelt und konfiguriert sind zum:

Verarbeiten der ein oder mehreren zeitveränderlichen Signale, um Signaturen für diese Signale zu entwickeln, wobei die Signaturen auf der Basis von linearen und nichtlinearen Charakteristiken der Signale, Phasen- und Zeitverzögerungen der Signale, Zeitfensterlängen der Signale, dem Grad der Kopplung zwischen verschiedenen physiologischen Signalen bestimmt werden, und
Berechnen der integrierten Wahrscheinlichkeit des Erfahrens des Zustands auf der Basis der entwickelten Signaturen;

wobei der zentrale Server (210) ferner zum Bereitstellen der integrierten Wahrscheinlichkeit und/oder der Signaturen der Person und/oder dem Trainings- oder Gesundheitsdienstleister für die Person und zum Aktualisieren der angezeigten integrierten Wahrscheinlichkeit und/oder der Signaturen nach einem Zeitplan konfiguriert ist.

12. Verfahren (300) nach Anspruch 8, das ferner Folgendes beinhaltet:

a. Sammeln von zeitvariierenden physiologischen Signalen für jede Person der Population, wobei die Population Personen, die ein(en) prädiktives/n Ereignis oder Zustand erfahren haben, und Personen umfasst, die das/den prädiktive/n Ereignis oder Zustand nicht erfahren haben;
b. Verarbeiten der zeitveränderlichen physiologischen Signale zum Erzeugen von Signaturprofilen für jede Person;
c. Vergleichen von Signaturprofilen für die Personen, die das/den prädiktive/n Ereignis oder Zustand erlebt haben, mit Signaturprofilen für die Personen, die das/den prädiktive/n Ereignis oder Zustand nicht erfahren haben, um ein korrelatives Muster zu identifizieren, das Signaturprofile von Personen, die das Ereignis oder den Zustand erfahren haben, und Personen unterscheidet, die das Ereignis oder den Zustand nicht erfahren haben;
d. Berechnen der integrierten Wahrscheinlichkeit des Ereignisses oder Zustands für einen Kandidaten durch Sammeln von zeitveränderlichen physischen Signalen für den Kandidaten, Verarbeiten der zeitveränderlichen physiologischen Signale zum Erzeugen von Signaturprofilen für den Kandidaten, und Identifizieren, ob das korrelative Muster in den Signaturprofilen für den Kandidaten erscheint.

13. Verfahren (300) nach Anspruch 12, wobei das Ereignis oder der Zustand Mortalität und/oder Auftreten von Krankheitssymptomen ist.

14. Verfahren (300) nach Anspruch 13, wobei das korrelative Muster eines oder mehrere von Signalveränderung bei Schlafbeginn oder bei einem Schlafzustandsübergang, Einnahme oder Nichteinnahme eines Medikaments oder Arzneimittels, Exposition oder Nichtexposition gegenüber einer Umweltbedingung oder einem Agens umfasst.

15. Verfahren (300) nach Anspruch 13, wobei die zeitveränderlichen Signale eine oder mehrere telemetrisch überwachte, nicht digitalisierte zeitveränderliche Signaturen umfassen.

**Revendications**

1. Procédé (300) mis en oeuvre par ordinateur pour évaluer la probabilité qu'un individu souffre d'un problème de santé, le procédé (300) comprenant :

   a. la fourniture (302) d'un ou plusieurs signaux variant dans le temps obtenus par la surveillance continue de l'individu ou de son environnement, les un ou plusieurs signaux variant dans le temps comprenant un ou plusieurs signaux physiologiques ;
   b. le traitement (306) des signaux pour développer des signatures pour ces signaux, les signatures étant déterminées sur la base de caractéristiques linéaires et non linéaires des signaux, de décalages de phase et de temps des signaux, de longueurs de fenêtres temporelles des signaux, du degré de couplage entre différents signaux physiologiques ;
   c. le calcul (320) d'une probabilité intégrée de souffrir du problème de santé sur la base des signatures développées ;
   d. la fourniture de la probabilité intégrée et/ou des signatures à l'individu et/ou à un prestataire d'entraînement ou de soins de santé pour l'individu ; et
   e. la mise à jour de la probabilité intégrée et/ou des signatures intégrées selon un horaire.

2. Procédé (300) selon la revendication 1, dans lequel le calcul d'une probabilité intégrée comprend l'analyse dynamique non linéaire d'un signal non linéaire dérivé d'au moins un des signaux variant dans le temps après discernement de signaux de bonne foi du bruit et de données manquantes via un procédé d'intelligence artificielle.

3. Procédé selon la revendication 1, dans lequel les signaux variant dans le temps comprennent des signaux physiologiques et/ou environnementaux continus variant dans le temps, et dans lequel les signatures sont des données de forme d'onde dérivées par télémétrie.

4. Procédé (300) selon la revendication 3, dans lequel les signaux comprennent une sortie d'onde continue obtenue à partir d'au moins : un électrocardiogramme (ECG), un électroencéphalogramme (EEG), un électrooculogramme (EOG), un électromyogramme (EMG), des excursions thoraciques et/ou abdominales enregistrées par des bandes de pléthysmographie inductives ; des enregistrements de débit d'air nasopharyngé, d'oxymétrie de pouls, de pression intracardiaque, d'électrogramme endocavitaire (EGM), de température, de position du corps détectée par une jauge et/ou une caméra, un moniteur de sommeil/activité, une surveillance acide/basique, un moniteur de respiration, d'accélération ou de marche, un moniteur d'exposition à la lumière ambiante, un moniteur d'exposition au bruit ambiant, un moniteur de température ambiante, des lectures de pression artérielle invasives et/ou non invasives, un glucomètre, un moniteur de saturation en oxygène de l'hémoglobine, un moniteur de pression partielle d'oxygène et de dioxyde de carbone du sang artériel, un moniteur de saturation en oxygène de l'hémoglobine tissulaire, un débit d'air nasopharyngé, et un moniteur d'activité métabolique basé sur une ou plusieurs mesures de $CO_2$ expiré, d'$O_2$ inspiré, de fréquence respiratoire, de volume respiratoire et de sang de laboratoire, comportant, sans s'y limiter, des mesures de numération plasmatique et de concentration de densité lipidique.

5. Procédé (300) selon la revendication 1, dans lequel au moins un des signaux associés au problème de santé comprend en outre un rapport subjectif individuel, familial et/ou de prestataire de soins de santé d'un état relatif du problème de santé de l'individu.

6. Procédé (300) selon la revendication 1, dans lequel les un ou plusieurs signaux comprennent au moins une signature subclinique.

7. Procédé selon la revendication 1, dans lequel l'individu est asymptomatique pour le problème de santé.

8. Procédé (300) selon la revendication 1, dans lequel les une ou plusieurs signatures sont dérivées d'une population d'individus manifestant le problème de santé ou de l'individu ou des deux.

9. Procédé (300) selon la revendication 1, dans lequel les signaux représentent une surveillance continue sur une période de temps d'une heure à plusieurs jours, et dans lequel la probabilité et les signatures intégrées sont mises à jour selon un horaire de 1 à 30 minutes.

10. Procédé (300) selon la revendication 1, comprenant en outre la création d'un profil de l'individu comprenant un composite des signatures variant dans le temps de l'individu et de la probabilité intégrée de groupes d'individus présentant des signatures similaires variant dans le temps, le stockage du profil de signature, et l'utilisation du profil de signature stocké lors du traitement ou de l'entraînement ultérieur de l'individu.

11. Système (200) d'évaluation de la probabilité qu'un individu souffre d'un problème de santé selon l'une quelconque des revendications de procédé précédentes, le système (200) comprenant :

a. un moyen de collecte de données (202) configuré pour fournir un ou plusieurs signaux variant dans le temps obtenus par la surveillance continue de l'individu ou de son environnement, les un ou plusieurs signaux variant dans le temps comprenant un ou plusieurs signaux physiologiques ;

b. un serveur central (210) configuré pour recevoir les un ou plusieurs signaux variant dans le temps ;

c. un ou plusieurs serveurs (214) couplés au serveur central (210) et configurés pour :

traiter les un ou plusieurs signaux variant dans le temps pour développer des signatures pour ces signaux, les signatures étant déterminées en fonction de caractéristiques linéaires et non linéaires des signaux, de décalages de phase et de temps des signaux, de longueurs de fenêtres temporelles des signaux, du degré de couplage entre différents signaux physiologiques, et
calculer la probabilité intégrée de souffrir du problème de santé en fonction des signatures développées ;

dans lequel le serveur central (210) est configuré en outre pour fournir la probabilité et/ou les signatures intégrées à l'individu et/ou au prestataire d'entraînement ou de soins de santé pour l'individu, et mettre à jour la probabilité et/ou les signatures intégrées affichées selon un planning.

12. Procédé (300) selon la revendication 8, comprenant en outre :

a. la collecte de signaux physiologiques variant dans le temps pour chaque individu de la population, dans lequel la population comprend des individus ayant subi un événement ou problème de santé prédictif et des individus qui n'ont pas subi l'événement ou le problème de santé prédictif ;

b. le traitement des signaux physiologiques variant dans le temps pour produire des profils de signature pour chaque individu ;

c. la comparaison des profils de signature des individus qui ont subi l'événement ou le problème de santé prédictif à des profils de signature des individus qui n'ont pas souffert de l'événement ou du problème de santé prédictif afin d'identifier un modèle corrélatif distinguant les profils de signature des individus qui ont souffert de l'événement ou du problème de santé et les individus qui n'ont pas souffert de l'événement ou du problème de santé ;

d. le calcul de la probabilité intégrée de l'événement ou du problème de santé pour un candidat en collectant des signaux physiques variant dans le temps du candidat, traitant les signaux physiologiques variant dans le temps pour produire des profils de signature pour le candidat et identifiant que le modèle corrélatif apparaît ou non dans les profils de signature du candidat.

13. Procédé (300) selon la revendication 12, dans lequel l'événement ou le problème de santé est la mortalité ou l'apparition de symptômes de maladie ou les deux.

14. Procédé (300) selon la revendication 13, dans lequel le modèle corrélatif comprend au moins une variation de signal durant un début d'endormissement ou une transition de l'état de sommeil, une utilisation ou non-utilisation d'un médicament ou d'un produit pharmaceutique, une exposition ou non-exposition à une condition ou agent environnemental.

15. Procédé (300) selon la revendication 13, dans lequel les signaux variant dans le temps comprennent une ou plusieurs signatures variant dans le temps non numérisées surveillées par télémétrie.

FIG. 1

FIG. 2

FIG. 3

MORTALITY RISK = $\mathbb{P}(\tau_F < T)$

i.e. probablity that $\tau_F$, estimated time of the first event within the next interval of time $T$ (and is that no such occurance is predicted), occurs before the time $T$, the prediction interval on interest.

$\tau_F = \min\{\tau_{F,i}\}_{i=1}^4$, the first occurance of an event, is estimated using a nonlinear regression model with variables given by the time series data and their derived features and ancillary data.

CARDIAC ARREST

SEVERE HEMORRHAGE

RESPIRATORY FAILURE

CIRCULATORY COLLAPSE

T=12h
T=9h
T=6h

95%

FIG. 4

**FIG. 5**

FIG. 6

FIG. 7

FIG. 8

**Derivation of Mean Volume Pulse Amplitude**

Mean Volume Pulse Amplitude=
Area Under contour/Duration of DVP Pulse

Peak of DVP Pulse

R

S

T=Duration of Pulse A

**FIG. 9**

**FIG. 10A**

**FIG. 10B**

**FIG. 10C**

CONTINUOUS PHYSIOLOGICAL WAVEFORMS (e.g. ECG,PPG) — *1100*

AI ANALYSIS TO DISCERN SIGNALS (e.g., NOISE, MISSING DATA, BONAFIDE DATA, UNIQUE FEATURES) IN AN INDIVIDUAL — *1102*

AI BASED "DEEP" LEARNING

*1104*

EXTRACT FEATURES FROM BONAFIDE SIGNALS (e.g., RR, QT, etc. FROM ECG)

CONSTRUCT A TIME SERIES FOR EACH EXRTRACTED FEATURE (e.g. RR FROM ECG) IN TIME BINS (e.g., 5-MIN) — *1106*

PERFORM NUMERICAL ANALYSIS ON EACH BIN OF EACH TIME SERIES (e.g. $SDNN_{RR}$, $ENTROPYX_{RR}$, $DFA_{RR}$, etc.) — *1108*

QUANTIFY THE TIME COURSE OF BINNED RESULTS FROM EACH NUMERICAL ANALYSIS (e.g. TRENDS, VARIATION AND VALUES OF $ENTROPYX_{RR}$, AT DIFFERENT TIMES) — *1110*

CONSTRUCT AN ARRAY OF RESULTS FROM THE CALCULATED PARAMETERS (e.g. $ENTROPYX_{RR}$, VALUES, TRENDS AND VARIATION) — *1112*

AI BASED "DEEP" LEARNING

*1114*

IDENTIFY THE MOST SIGNIFICANT INDEPENDENT PREDICTORS OVER THE COURSE OF AN INDIVIDUAL'S HEALTH, ILLNESS, AND RESPONSE TO TREATMENTS

CONSTRUCT AND STORE PERSONLIZED MULTIVARIATE PREDICTIVE MODELS FOR GROUPS OF INDIVIDUALS AND FOR EACH INDIVIDUAL — *1116*

**FIG. 11**

FIG. 12C

FIG. 12B

FIG. 12A

FIG. 13

**FIG. 14**

FIG. 15

**FIG. 16**

**FIG. 17**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013040751 W
- US 2013040677 W
- US 20150133795 A, Gordon F. Tomaselli

- WO 2013003787 A2
- US 2015133795 A1

**Non-patent literature cited in the description**

- **KNAUS, W.A et al.** The APACHE III prognostic system: Risk prediction of hospital mortality for critically ill hospitalized adults. *Chest,* 1991, vol. 100, 1619-1636

- **LE GALL, J.R. ; LEMESHOW, S. ; SAULNIER, F.** A new Simplified Acute Physiology Score (SAPS II) based on a European/North American multicenter study. *JAMA,* 1993, vol. 270, 2957-2963